# EUROPEAN PATENT APPLICATION

(11) **EP 4 434 371 A1**
(43) Date of publication of application: **25.09.2024**
(21) Application number: 21964776.5
(22) Date of filing: 19.11.2021
(51) Int. Cl.: A24F 40/53, A24F 40/57

(54) **CIRCUIT UNIT FOR AEROSOL GENERATION DEVICE, AEROSOL GENERATION DEVICE, AND PROGRAM**

(71) Applicant: Japan Tobacco Inc., Tokyo 105-6927 (JP)
(72) Inventor: NAKANO, Takuma, Tokyo 130-8603 (JP); MIZUGUCHI, Kazuma, Tokyo 130-8603 (JP)
(74) Representative: Hoffmann Eitle
(86) International application number: PCT/JP2021/042553
(87) International publication number: WO 2023/089761

(57) **Abstract**

This circuit unit for an aerosol generation device is provided with a control part that controls supply of electric power to a load for heating an aerosol source. When the remaining quantity of the aerosol source is lower than a first remaining quantity, the control part sets a first period, which is to be utilized for determining whether the length of an interval between the most recent sucking and the current sucking of aerosol is long or not, to a value longer than a reference value.

## Description

### Technical Field

The present invention relates to a circuit unit for an aerosol generation device, an aerosol generation device, and a program.

### Background Art

In an aerosol generation device that generates an aerosol by heating a liquid including, for example, a flavoring agent, energization of a heater starts in response to sensing of an inhalation action by the user, and a liquid in a glass fiber called a wick is atomized (becomes an aerosol). The aerosol is generated in response to the temperature of the liquid in the wick reaching the boiling point.

### Citation List

### Patent Literature

PTL 1: US Patent Application Publication No. 2020/0329776

### Summary of Invention

### Technical Problem

For the aerosol generation device, the energizing time of the heater is designed while a typical inhalation action is assumed, however, when an inhalation action in which the interval between inhalation (puff) and inhalation (puff) (hereinafter also referred to as "puff interval") is shorter than in the typical inhalation action is repeated, heating of the liquid is started before the liquid temperature in the wick drops to a sufficiently low temperature. When the liquid temperature at the start of energization is high, vaporization of the liquid is accelerated. As a result, the amount of consumption of the liquid after the start of energization becomes higher than in the typical inhalation action.

Meanwhile, supply of the liquid to the wick depends on a capillary action, and the speed of liquid sending by the capillary action depends on the amount of residual liquid. Therefore, in a situation in which the liquid supply speed decreases due to a decrease in the amount of residual liquid, the amount of aerosol that can be generated during a single puff becomes smaller than in a case where the amount of residual liquid is large. That is, a sufficient aerosol is no longer generated during the single puff.

When a threshold used in a determination of a short puff interval is the same regardless of the amount of residual liquid, heating of the liquid continues even in a state in which the liquid supply speed decreases, and an event the same as drying up occurs.

The present invention provides a technique for suppressing drying up during inhalation when the residual amount of an aerosol source in an aerosol generation device is small.

### Solution to Problem

An invention according to claim 1 is a circuit unit for an aerosol generation device, including:
a controller that controls supply of electric power to a load that heats an aerosol source, in which when a residual amount of the aerosol source is smaller than a first residual amount, the controller sets a first period that is used to determine whether a length of an interval between a preceding instance of aerosol inhalation and a present instance of aerosol inhalation is long or short to a value longer than a reference value.

An invention according to claim 2 is the circuit unit for an aerosol generation device according to claim 1, in which the controller computes the residual amount of the aerosol source by calculation and controls a length of the first period on the basis of the computed residual amount.

An invention according to claim 3 is the circuit unit for an aerosol generation device according to claim 1, further including: a first sensor that detects the residual amount of the aerosol source, in which the controller controls a length of the first period on the basis of the residual amount detected by the first sensor.

An invention according to claim 4 is the circuit unit for an aerosol generation device according to claim 1, in which when a number of times an instance of inhalation whose inter-aerosol-inhalation interval is shorter than the first period consecutively appears exceeds a first number of times, the controller controls the first period that is used in a next instance of inhalation and beyond so as to be longer in a step-by-step manner as the number of times increases.

An invention according to claim 5 is the circuit unit for an aerosol generation device according to claim 1, in which when the residual amount of an aerosol is smaller than the first residual amount, the controller controls the first period so as to be a time longer than an interval of an immediately preceding instance.

An invention according to claim 6 is the circuit unit for an aerosol generation device according to claim 1, in which when the aerosol source is heated at a second temperature lower than a first temperature prior to heating of the aerosol source at the first temperature, the heating involving aerosol generation, the controller controls the first period that is used when the residual amount of an aerosol is smaller than the first residual amount so as to be a value smaller than the first period that is used only in a case of heating involving aerosol generation and when the residual amount of the aerosol is smaller than the first residual amount.

An invention according to claim 7 is the circuit unit for an aerosol generation device according to claim 6, in which when a number of times an instance of inhalation whose inter-aerosol-inhalation interval is shorter than the first period consecutively appears exceeds a first number of times, the controller controls the first period that is used in a next instance of inhalation and beyond so as to be longer in a step-by-step manner as the number of times increases.

An invention according to claim 8 is the circuit unit for an aerosol generation device according to claim 6, in which when the residual amount of an aerosol is smaller than the first residual amount, the controller controls the first period so as to be a time longer than an interval of an immediately preceding instance.

An invention according to claim 9 is the circuit unit for an aerosol generation device according to claim 6, in which when heating not involving aerosol generation is performed, the controller controls an amount of electric power that is supplied to the load for aerosol generation so as to be a value smaller than an amount of electric power that is supplied to the load when only heating involving aerosol generation is performed.

An invention according to claim 10 is the circuit unit for an aerosol generation device according to any one of claims 1 to 9, further including: a second sensor that detects a temperature of the load, in which at a time point when the temperature detected by the second sensor reaches a third temperature, the controller forcibly terminates heating by the load.

An invention according to claim 11 is the circuit unit for an aerosol generation device according to any one of claims 1 to 9, in which the controller further includes a third sensor that detects a temperature of the aerosol source, and at a time point when the temperature detected by the third sensor reaches a fourth temperature, the controller forcibly terminates heating by the load.

An invention according to claim 12 is the circuit unit for an aerosol generation device according to any one of claims 1 to 9, in which when an inter-aerosol-inhalation interval is shorter than the first period, the controller controls a first maximum voltage value of a voltage that is supplied to the load for aerosol generation so as to be a value smaller than a second maximum voltage value of a voltage that is supplied to the load when the inter-aerosol-inhalation interval is longer than a threshold.

An invention according to claim 13 is an aerosol generation device including: a controller that controls supply of electric power to a load that heats an aerosol source, in which when a residual amount of the aerosol source is smaller than a first residual amount, the controller sets a first period that is used to determine whether a length of an interval between a preceding instance of aerosol inhalation and a present instance of aerosol inhalation is long or short to a value longer than a reference value.

An invention according to claim 14 is a program for causing a computer that controls supply of electric power to a load that heats an aerosol source to implement: a function of, when a residual amount of the aerosol source is smaller than a first residual amount, setting a first period that is used to determine whether a length of an interval between a preceding instance of aerosol inhalation and a present instance of aerosol inhalation is long or short to a value longer than a reference value.

### Advantageous Effects of Invention

With the invention according to claim 1, drying up during inhalation when the residual amount of the aerosol source in the aerosol generation device is small can be suppressed.

With the invention according to claim 2, the number of sensors provided in the aerosol generation device can be made smaller.

With the invention according to claim 3, control based on the actual residual amount can be implemented.

With the invention according to claim 4, a determination that the inhalation interval is short can be more likely to be made as a state in which the inter-inhalation interval is short continues.

With the invention according to claim 5, a determination that the inhalation interval is short can be more likely to be made by making longer than the latest interval.

With the invention according to claim 6, when the aerosol source is heated prior to heating involving aerosol generation, the number of times a real heating time is shortened can be made smaller even when the residual amount of the aerosol source is small.

With the invention according to claim 7, a determination that the inhalation interval is short can be more likely to be made as a state in which the inter-inhalation interval is short continues.

With the invention according to claim 8, a determination that the inhalation interval is short can be more likely to be made by making longer than the latest interval.

With the invention according to claim 9, aerosol generation is accelerated by heating of the aerosol source prior to heating involving aerosol generation, and therefore, the amount of electric power that is supplied for aerosol generation can be reduced.

With the invention according to claim 10, drying up can be suppressed even when an environment in which drying up is likely to occur is detected.

With the invention according to claim 11, drying up can be suppressed even when an environment in which drying up is likely to occur is detected.

With the invention according to claim 12, drying up during inhalation when the residual amount of the aerosol source in the aerosol generation device is small can be suppressed.

With the invention according to claim 13, drying up during inhalation when the residual amount of the aerosol source in the aerosol generation device is small can be suppressed.

With the invention according to claim 14, drying up during inhalation when the residual amount of the aerosol source in the aerosol generation device is small can be suppressed.

### Brief Description of Drawings

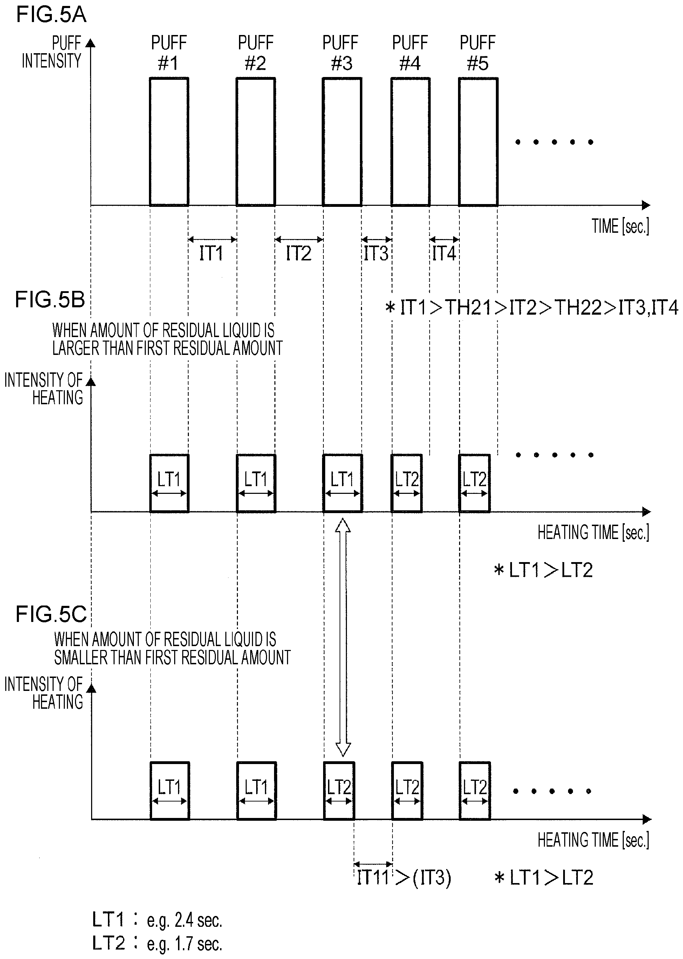

[Fig. 1] Fig. 1 is a diagram for explaining an example external configuration of an aerosol generation device assumed in Embodiment 1.
[Fig. 2] Fig. 2 is a diagram schematically illustrating an internal configuration of the aerosol generation device assumed in Embodiment 1.
[Fig. 3] Fig. 3 is a flowchart for explaining example control of a real heating time by a controller used in Embodiment 1.
[Fig. 4] Fig. 4 is a diagram for explaining example settings of a determination threshold in accordance with the amount of residual liquid.
[Figs. SAto 5C] Figs. 5A to 5C are diagrams for explaining a relationship between a puff interval and a setting of the real heating time in Embodiment 1. Fig. 5A illustrates example timings of inhalation, Fig. 5B illustrates example settings of the real heating time when the amount of residual liquid is larger than a first residual amount, and Fig. 5C illustrates example settings of the real heating time when the amount of residual liquid is smaller than the first residual amount.

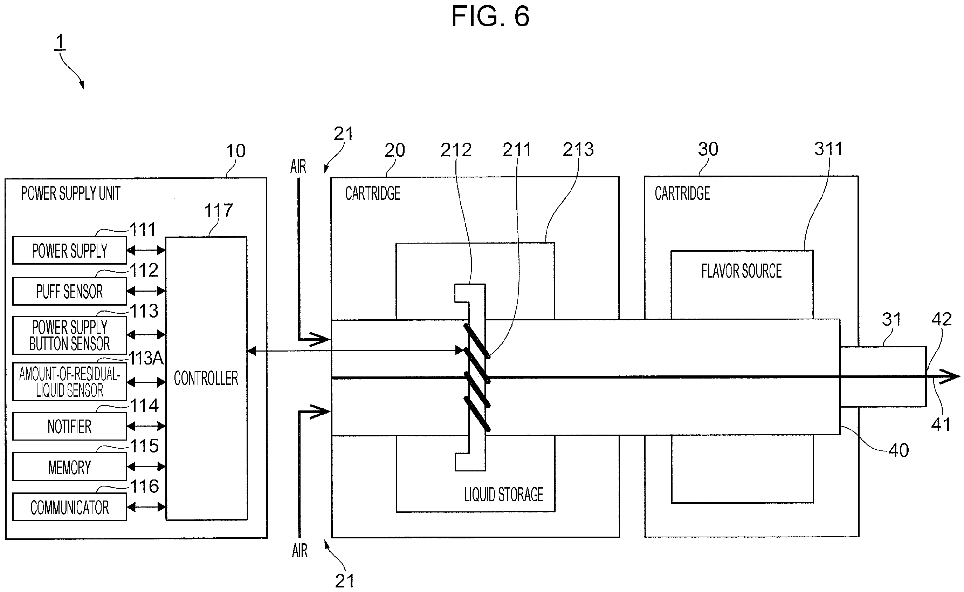

[Fig. 6] Fig. 6 is a diagram schematically illustrating an internal configuration of an aerosol generation device assumed in Embodiment 2.

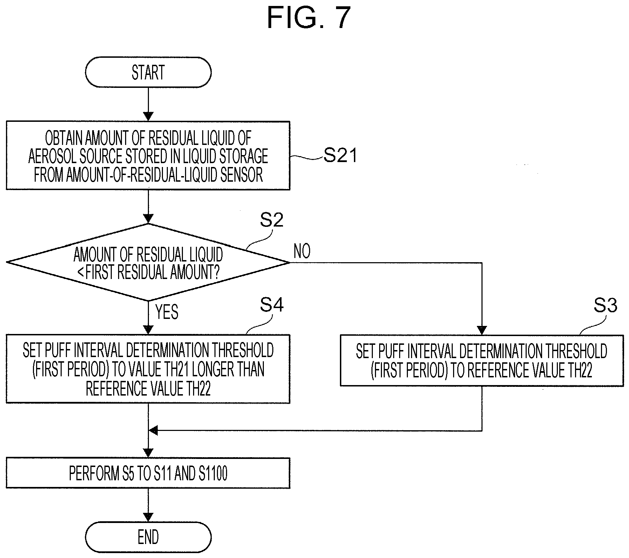

[Fig. 7] Fig. 7 is a flowchart for explaining example control of the real heating time by a controller used in Embodiment 2.

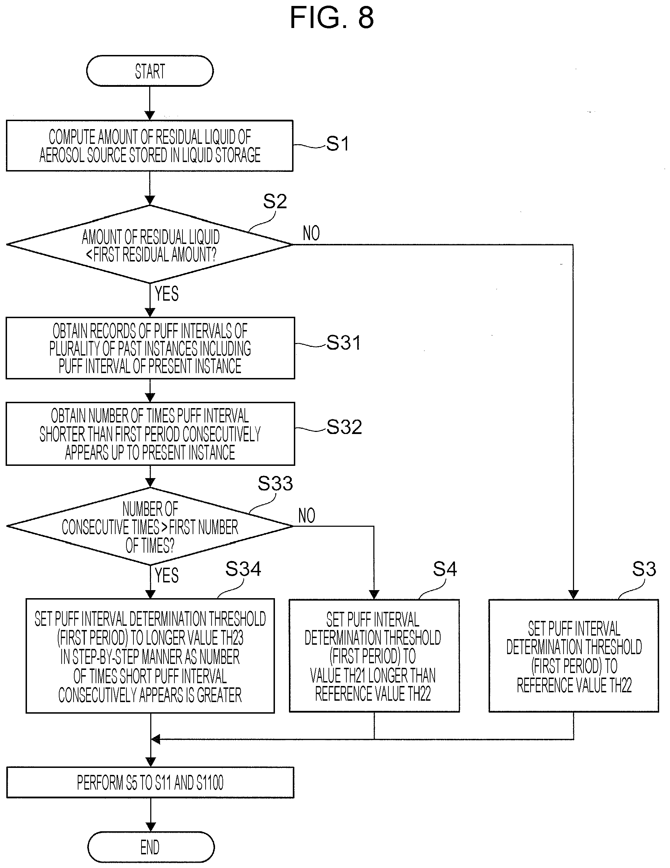

[Fig. 8] Fig. 8 is a flowchart for explaining example control of the real heating time by a controller used in Embodiment 3.

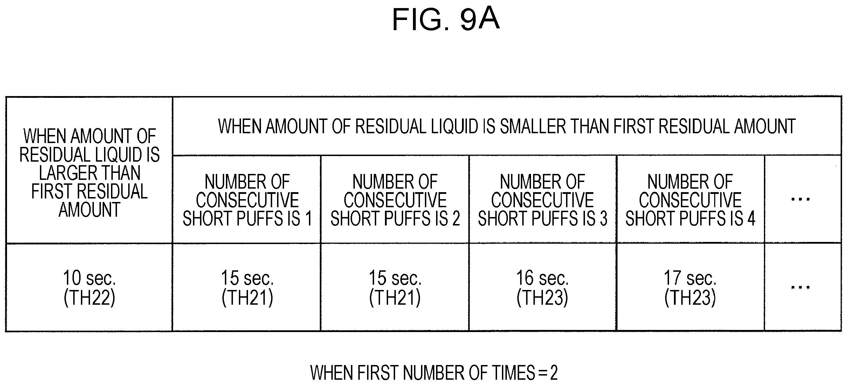

[Fig. 9A] Fig. 9A is a diagram for explaining example settings of a puff interval determination threshold in Embodiment 3.

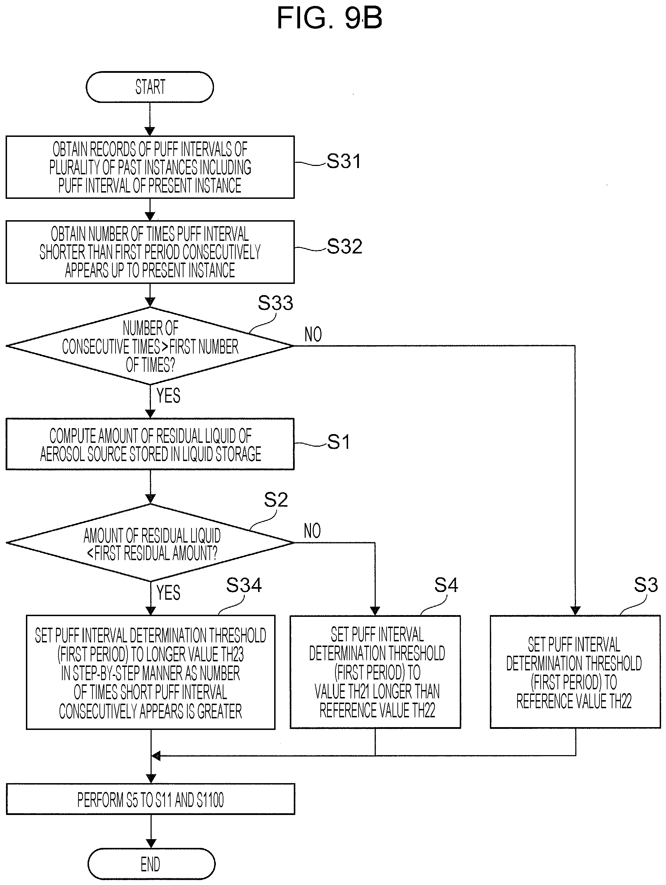

[Fig. 9B] Fig. 9B is a flowchart for explaining other example control in Embodiment 3.

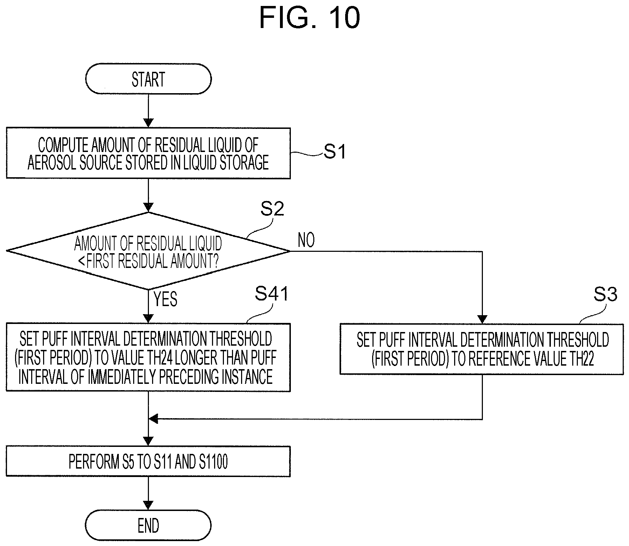

[Fig. 10] Fig. 10 is a flowchart for explaining example control of the real heating time by a controller used in Embodiment 4.

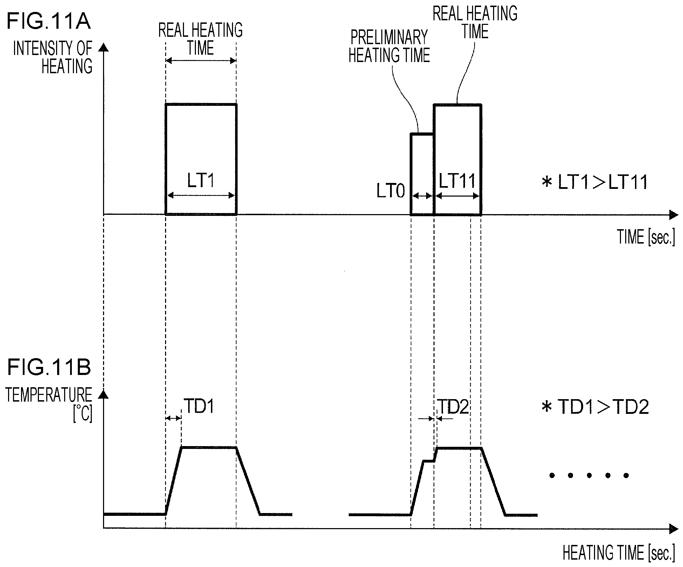

[Figs. 11A and 11B] Figs. 11A and 11B are diagrams for explaining a preliminary heating time. Fig. 11A illustrates a relationship between the positions of the preliminary heating time and the real heating time and Fig. 11B illustrates changes in the temperature of an aerosol source.

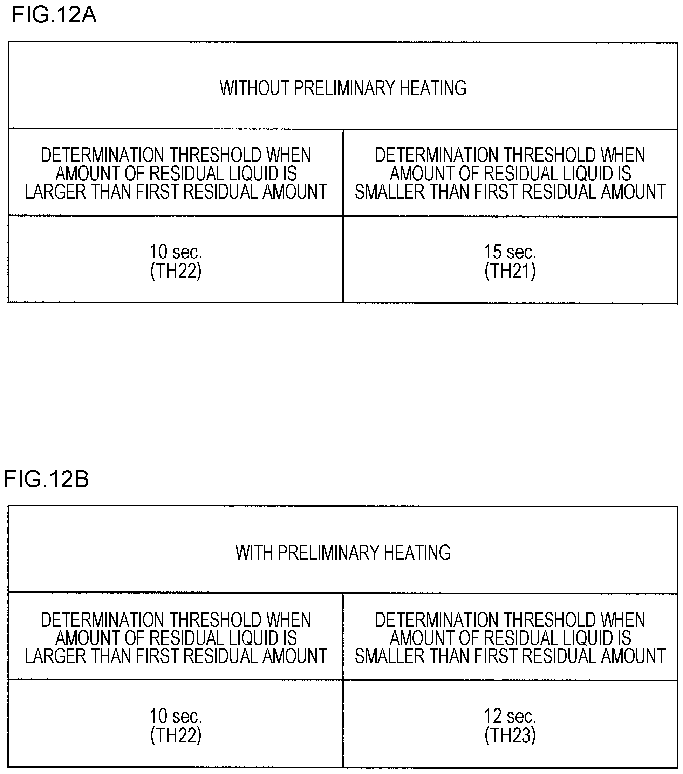

[Figs. 12A and 12B] Figs. 12A and 12B are diagrams for explaining example settings of the determination threshold in accordance with whether preliminary heating is used and the amount of residual liquid. Fig. 12A illustrates a case without preliminary heating and Fig. 12B illustrates a case with preliminary heating.

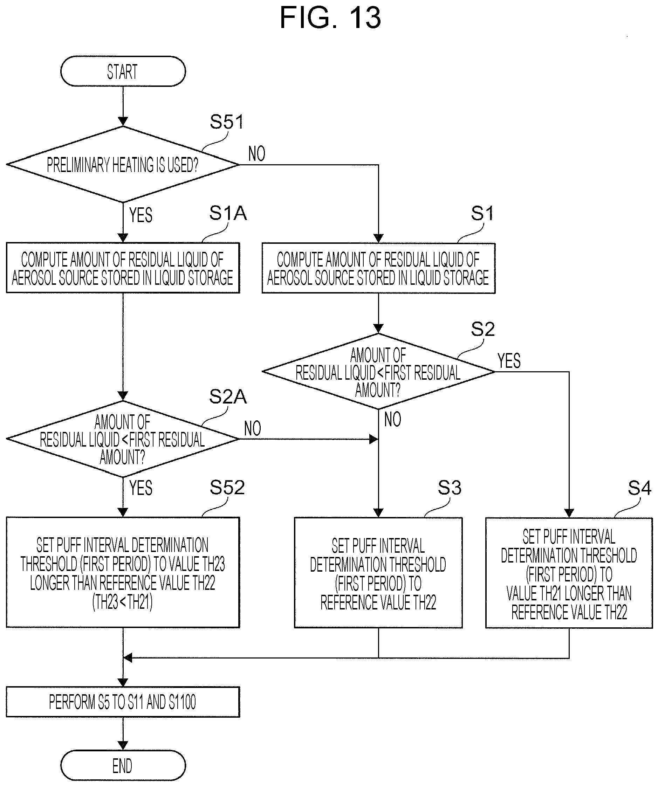

[Fig. 13] Fig. 13 is a flowchart for explaining example control of the real heating time by a controller used in Embodiment 5.

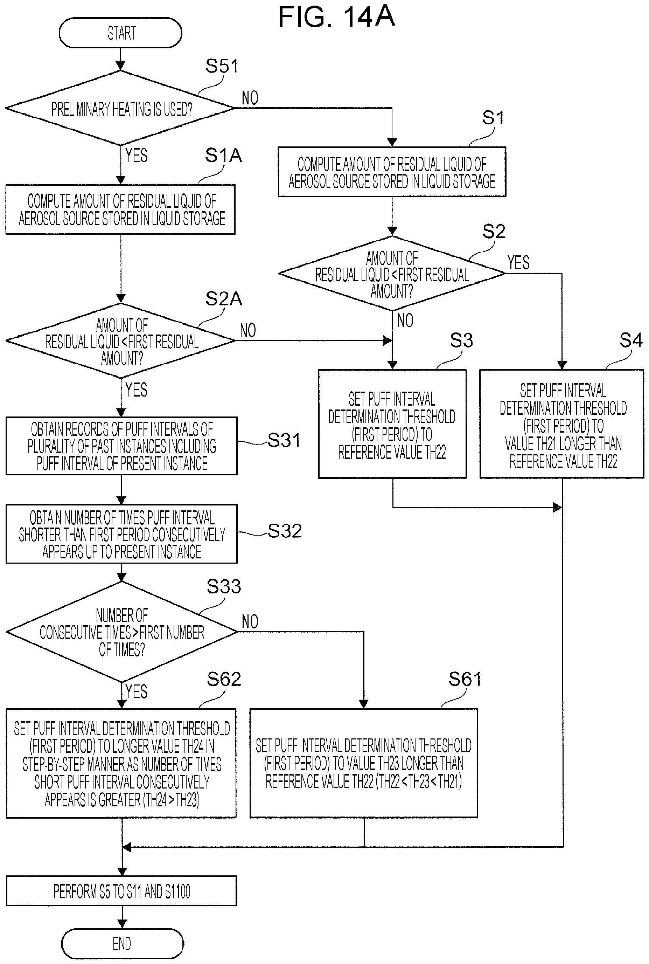

[Fig. 14A] Fig. 14A is a flowchart for explaining example control of the real heating time by a controller used in Embodiment 6.

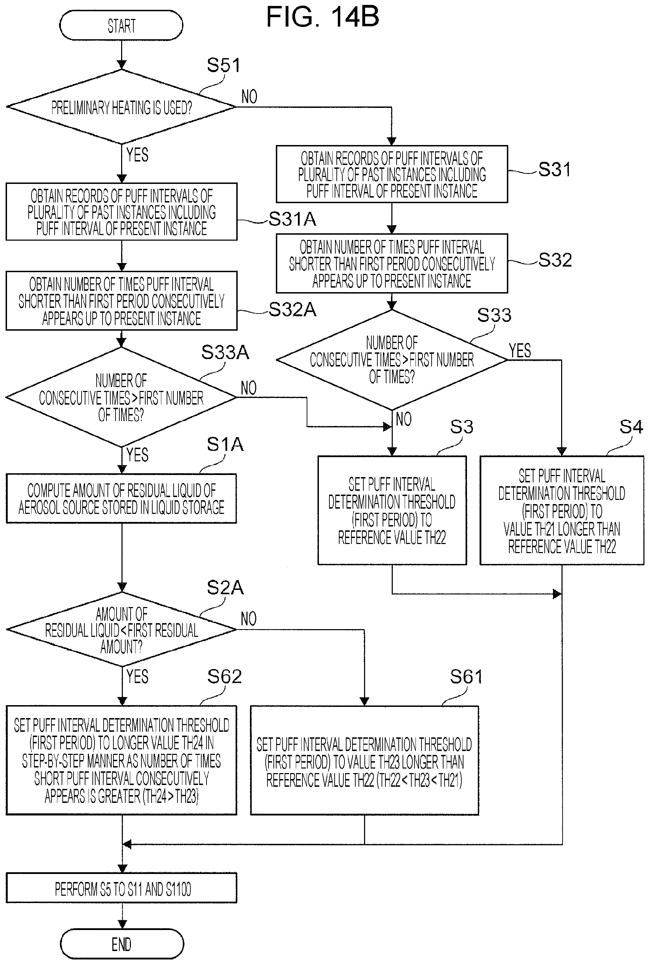

[Fig. 14B] Fig. 14B is a flowchart for explaining other example control 1 in Embodiment 6.

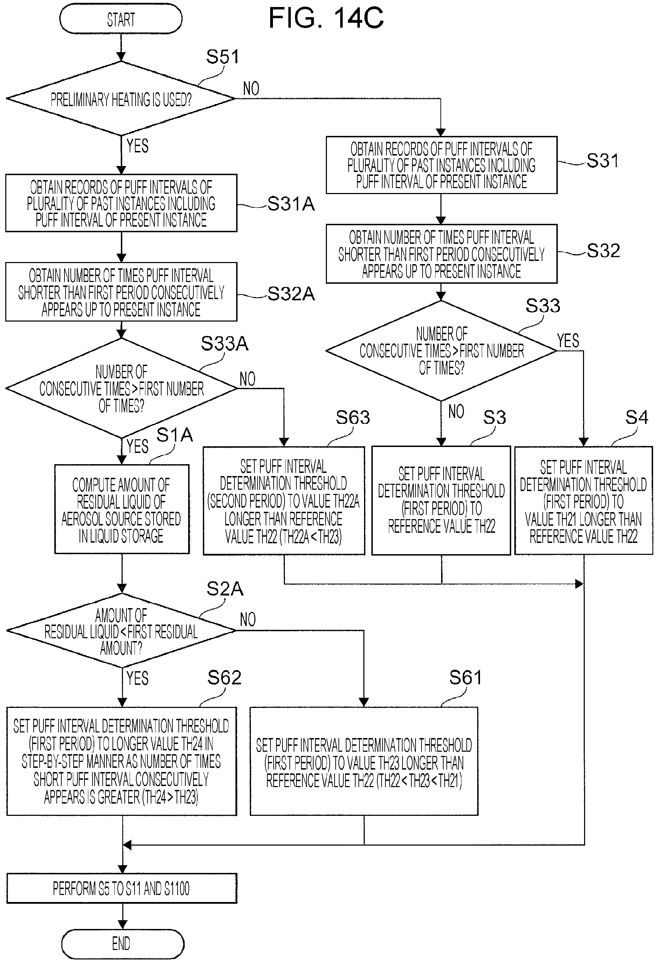

[Fig. 14C] Fig. 14C is a flowchart for explaining other example control 2 in Embodiment 6.

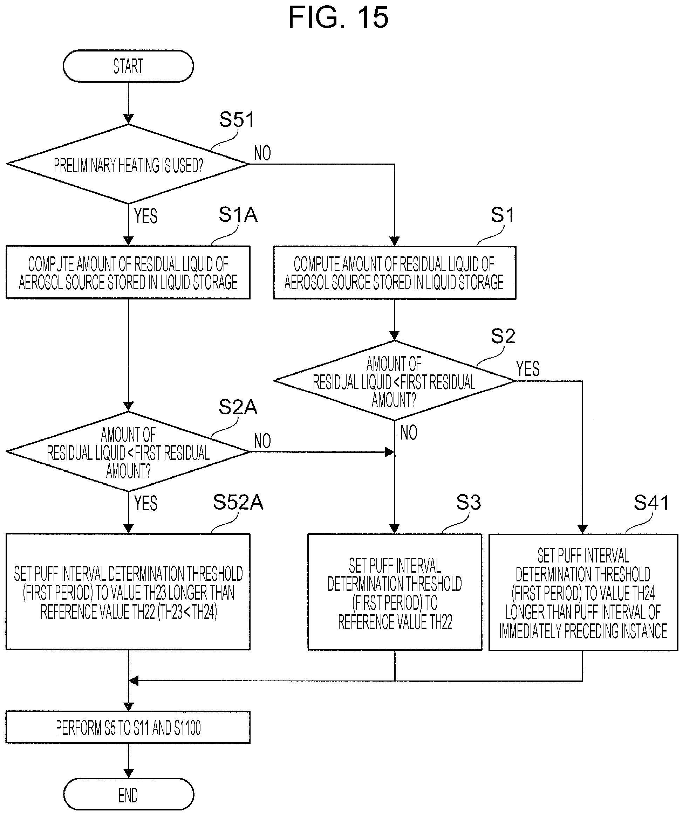

[Fig. 15] Fig. 15 is a flowchart for explaining example control of the real heating time by a controller used in Embodiment 7.

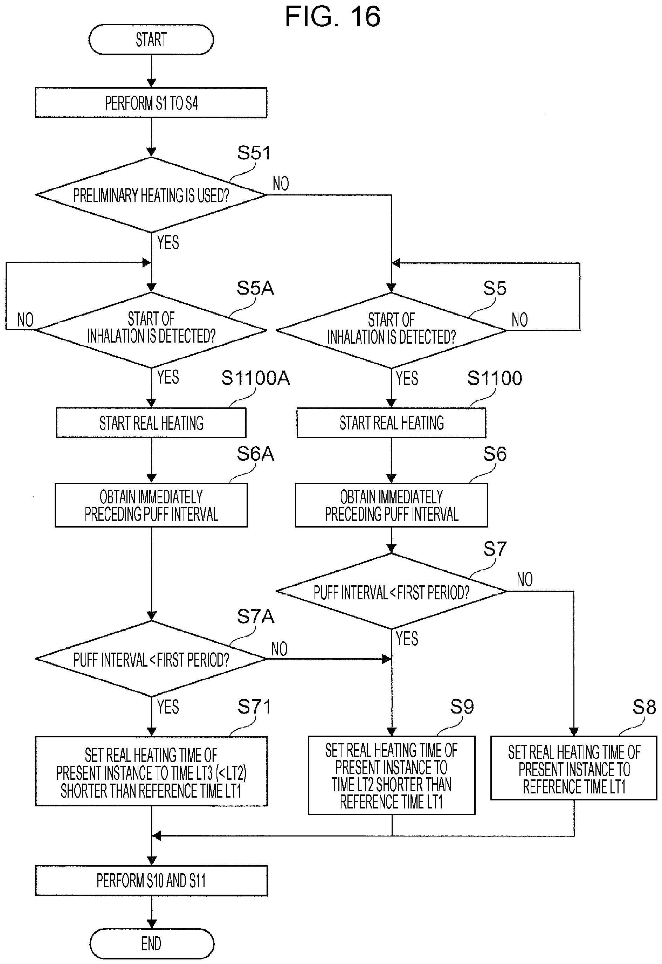

[Fig. 16] Fig. 16 is a flowchart for explaining example control of the real heating time by a controller used in Embodiment 8.

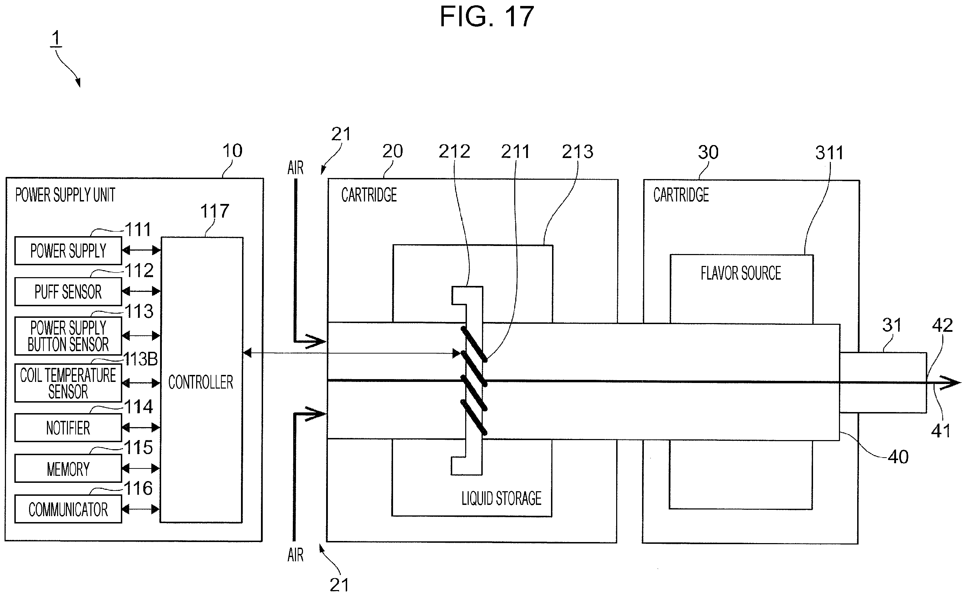

[Fig. 17] Fig. 17 is a diagram schematically illustrating an internal configuration of an aerosol generation device assumed in Embodiment 9.

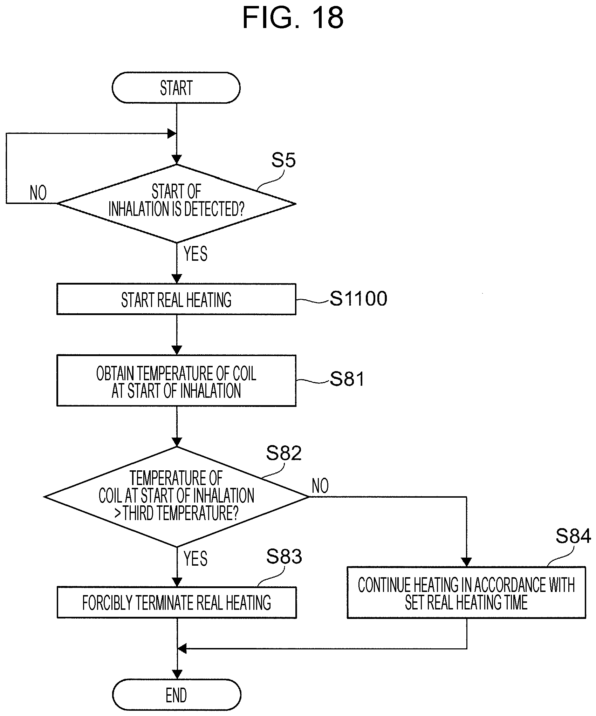

[Fig. 18] Fig. 18 is a flowchart for explaining example control of the real heating time by a controller used in Embodiment 9.

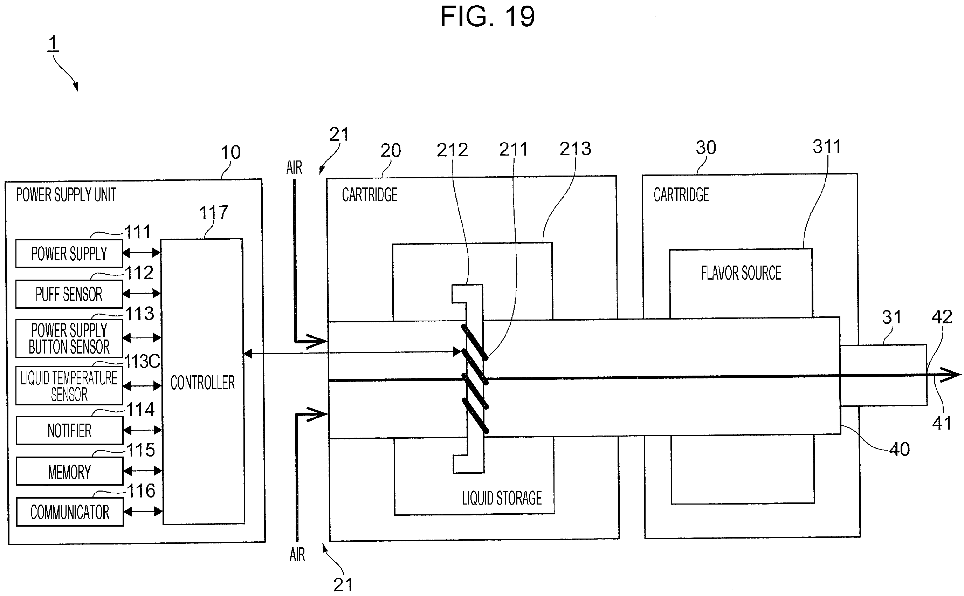

[Fig. 19] Fig. 19 is a diagram schematically illustrating an internal configuration of an aerosol generation device assumed in Embodiment 10.

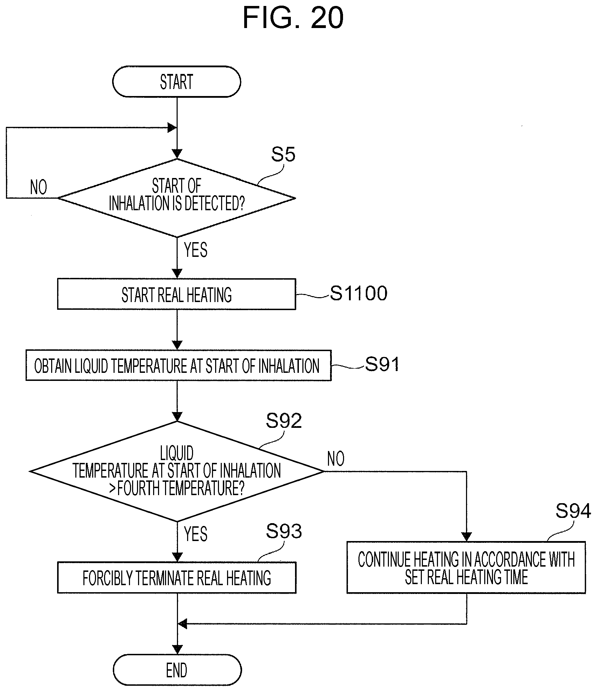

[Fig. 20] Fig. 20 is a flowchart for explaining example control of the real heating time by a controller used in Embodiment 10.

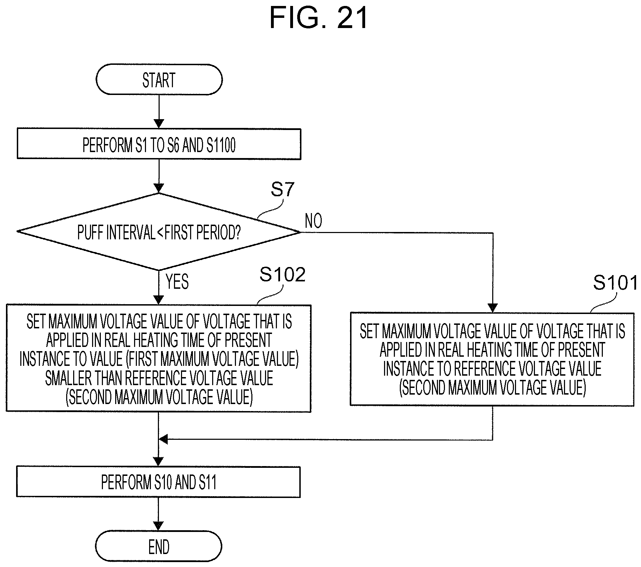

[Fig. 21] Fig. 21 is a flowchart for explaining example control of the real heating time by a controller used in Embodiment 11.

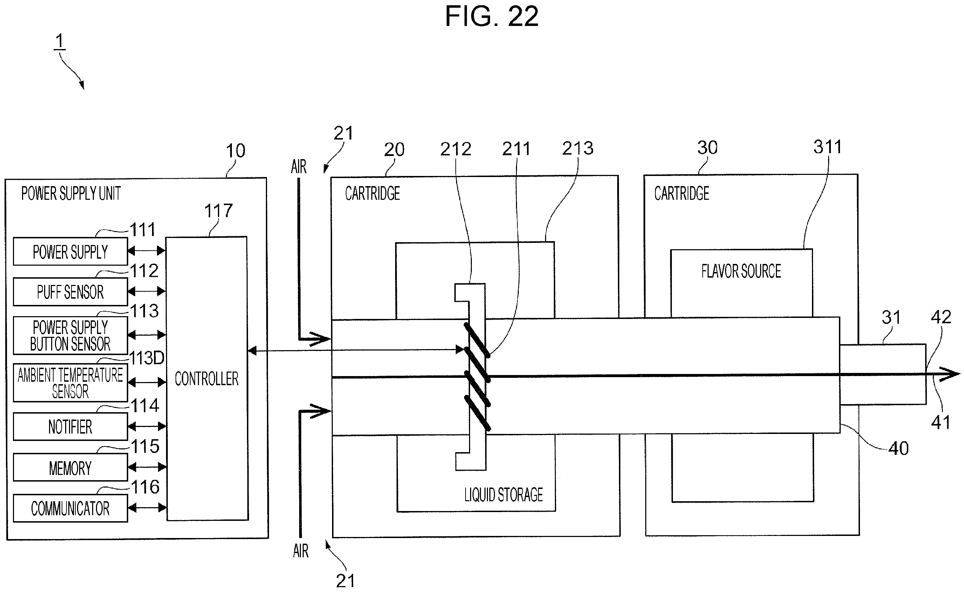

[Fig. 22] Fig. 22 is a diagram schematically illustrating an internal configuration of an aerosol generation device assumed in Embodiment 12.

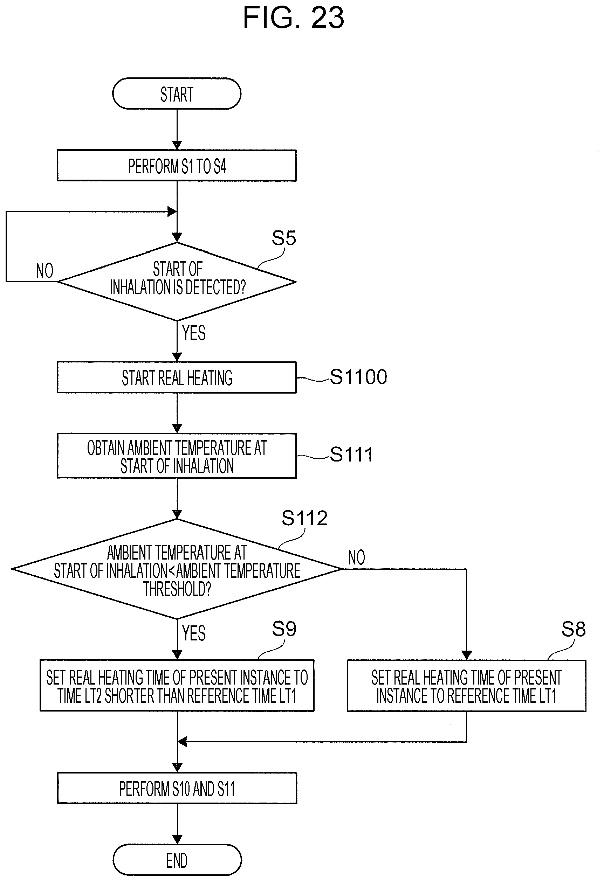

[Fig. 23] Fig. 23 is a flowchart for explaining example control of the real heating time by a controller used in Embodiment 11.

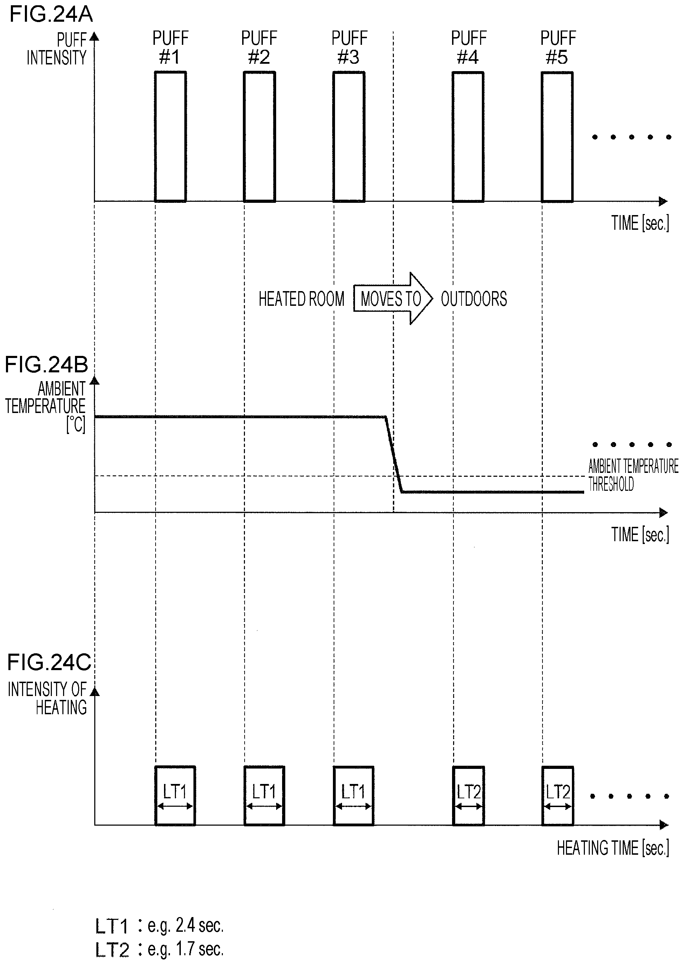

[Figs. 24A to 24C] Figs. 24A to 24C are diagrams for explaining a relationship between a puff interval and a setting of the real heating time in Embodiment 12. Fig. 24A illustrates example timings of inhalation, Fig. 24B illustrates changes in an ambient temperature, and Fig. 24C illustrates example settings of the real heating time.

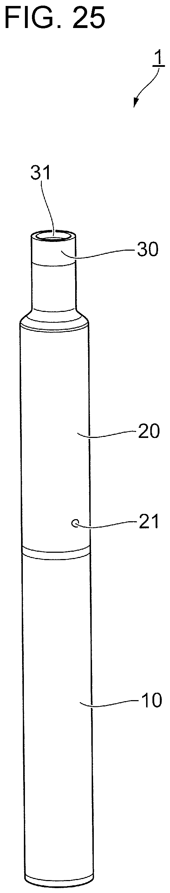

[Fig. 25] Fig. 25 is a diagram for explaining an example external configuration of an aerosol generation device assumed in Embodiment 13.

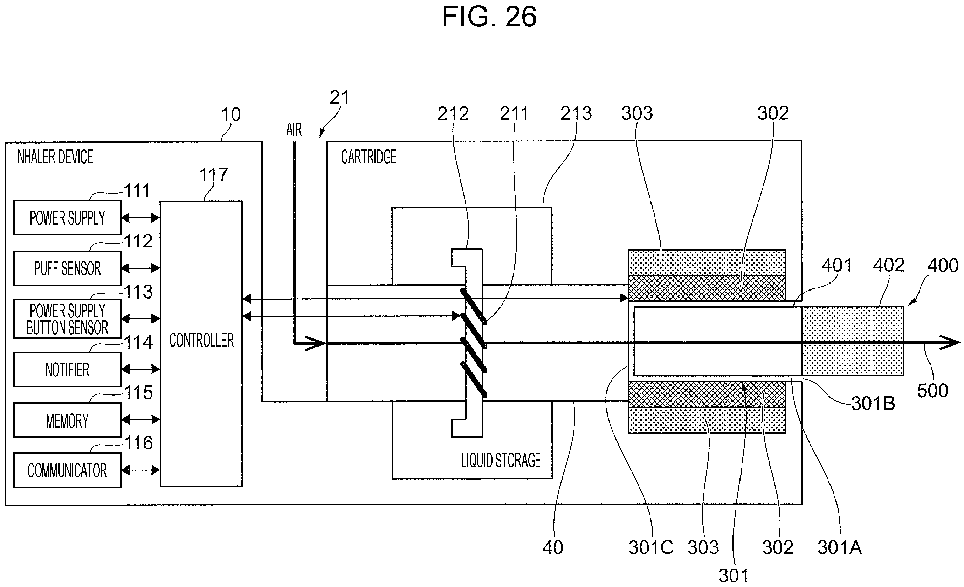

[Fig. 26] Fig. 26 is a diagram schematically illustrating an example internal configuration of an aerosol generation device assumed in Embodiment 14.

### Description of Embodiments

Embodiments of the present invention will be described below with reference to the drawings. The same parts in the drawings are assigned the same reference signs and illustrated.

### <Embodiment 1>

### <External Configuration>

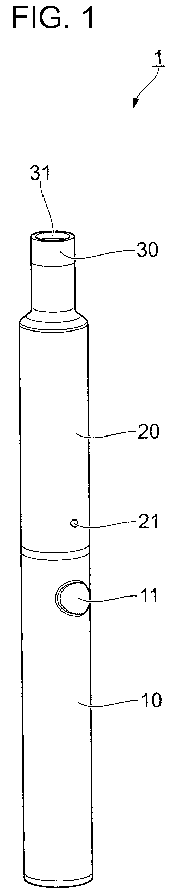

Fig. 1 is a diagram for explaining an example external configuration of an aerosol generation device 1 assumed in Embodiment 1.

The aerosol generation device 1 illustrated in Fig. 1 is one form of an electronic cigarette and generates an aerosol to which a flavor is imparted without combustion. The electronic cigarette illustrated in Fig. 1 has a substantially cylindrical form.

The aerosol generation device 1 illustrated in Fig. 1 is constituted by a plurality of units. In Fig. 1, the plurality of units are a power supply unit 10, a cartridge 20, which incorporates therein an aerosol source, and a cartridge 30, which incorporates therein a flavor source.

In this embodiment, the cartridge 20 is detachable from the power supply unit 10, and the cartridge 30 is detachable from the cartridge 20. In other words, both the cartridge 20 and the cartridge 30 are replaceable.

The power supply unit 10 incorporates therein an electronic circuit and so on. The power supply unit 10 is one form of the circuit unit. On the side surface of the power supply unit 10, a power supply button 11 is provided. The power supply button 11 is an example of an operation unit used in input of a user instruction into the power supply unit 10.

The cartridge 20 incorporates therein a liquid storage that stores a liquid that is the aerosol source, a liquid guide that draws the liquid from the liquid storage by a capillary action, and a heater that heats and vaporizes the liquid held in the liquid guide.

On the side surface of the cartridge 20, an inlet hole for air (hereinafter referred to as "air inlet hole") 21 is provided. Air flowing in through the air inlet hole 21 passes through the inside of the cartridge 20 and is released from the cartridge 30. The cartridge 20 is also called an atomizer.

The cartridge 30 incorporates therein a flavor unit that imparts a flavor to an aerosol. The cartridge 30 is provided with an inhalation port 31.

### <Internal Configuration>

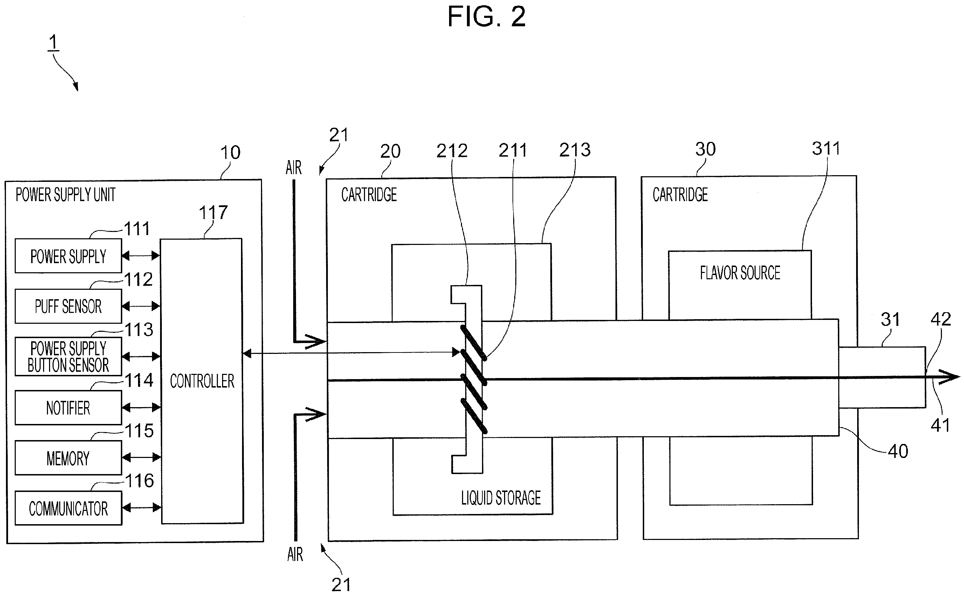

Fig. 2 is a diagram schematically illustrating an internal configuration of the aerosol generation device 1 assumed in Embodiment 1.

The aerosol generation device 1 is constituted by the power supply unit 10 and the cartridges 20 and 30.

The power supply unit 10 incorporates therein a power supply 111, a puff sensor 112, a power supply button sensor 113, a notifier 114, a memory 115, a communicator 116, and a controller 117.

The cartridge 20 incorporates therein a heater 211, a liquid guide 212, and a liquid storage 213.

The cartridge 30 incorporates therein a flavor source 311. One end of the cartridge 30 is used as the inhalation port 31.

Inside the cartridges 20 and 30, an airflow path 40 connected to the air inlet hole 21 is formed.

The power supply 111 is a device that stores electric power necessary for operations. The power supply 111 supplies electric power to each part that constitutes the aerosol generation device 1 through control by the controller 117. The power supply 111 is formed of, for example, a rechargeable battery, such as a lithium ion secondary battery.

The puff sensor 112 is a sensor that detects inhalation of an aerosol by the user and is formed of, for example, a flow sensor. The puff sensor 112 is an example of a first sensor.

The power supply button sensor 113 is a sensor that detects an operation performed on the power supply button 11 (see Fig. 1) and is formed of, for example, a pressure sensor. Note that the power supply unit 10 is provided with various sensors in addition to the puff sensor 112 and the power supply button sensor 113.

The notifier 114 is a device that is used in providing information to the user. Examples of the notifier 114 include a light-emitting device, a display device, a sound output device, and a vibration device.

The memory 115 is a device that stores various types of information necessary for operations of the aerosol generation device 1. As the memory 115, a non-volatile storage medium, such as a flash memory, is used.

The communicator 116 is a communication interface that is in conformity with a wired or wireless communication standard. As the communication standard, for example, Wi-Fi (registered trademark) or Bluetooth (registered trademark) is used.

The controller 117 is a device that functions as an arithmetic processing unit or a control device, and controls all operations in the aerosol generation device 1 through execution of various programs. The controller 117 is implemented as an electronic circuit, such as a CPU (central processing unit) or an MPU (micro-processing unit).

The liquid storage 213 is a tank that stores the aerosol source. When the aerosol source stored in the liquid storage 213 is atomized, an aerosol is generated.

As the aerosol source, a liquid, such as polyhydric alcohol, which is, for example, glycerine or propylene glycol, or water, is used. The aerosol source may include a flavor component derived from tobacco or not derived from tobacco.

When the aerosol generation device 1 is a medical inhaler, such as a nebulizer, the aerosol source may include medicine.

The liquid guide 212 is a member that guides the aerosol source, which is a liquid, from the liquid storage 213 to a heating region and holds the aerosol source. As the liquid guide 212, a member called a wick formed by twining a fiber material, such as a glass fiber, or a porous material, such as porous ceramic, is used. When the liquid guide 212 is formed of a wick, the aerosol source stored in the liquid storage 213 is guided to the heating region by a capillary action of the wick.

The heater 211 is a member that heats the aerosol source held in the heating region to atomize the aerosol source and generate an aerosol.

In Fig. 2, the heater 211 is a coil and is wound around the liquid guide 212. A region around which the coil is wound in the liquid guide 212 is the heating region. With heat produced by the heater 211, the temperature of the aerosol source held in the heating region rises to the boiling point and an aerosol is generated.

The heater 211 produces heat in response to supply of electric power from the power supply 111. Supply of electric power to the heater 211 starts when a predetermined condition is satisfied. Examples of the predetermined condition include the start of inhalation by the user, pressing of the power supply button 11 a specific number of times, and input of predetermined specific information. In this embodiment, supply of electric power to the heater 211 starts in response to detection of inhalation.

Supply of electric power to the heater 211 stops when a predetermined condition is satisfied. Examples of the predetermined condition include the end of inhalation by the user, the end of a real heating time described below, pressing and holding down of the power supply button 11, and input of predetermined specific information. In this embodiment, supply of electric power to the heater 211 stops in response to the end of inhalation.

The heater 211 here is an example of a load that consumes electric power.

The flavor source 311 is a structural element that imparts a flavor component to an aerosol generated inside the cartridge 20. The flavor source 311 includes a flavor component that is derived from tobacco or not derived from tobacco.

The airflow path 40, which penetrates the inside of the cartridge 20 and the cartridge 30, is a flow path for air and an aerosol that are inhaled by the user. The airflow path 40 has a tubular structure that has the air inlet hole 21 as an inlet of air and an air outlet hole 42 as an outlet of air.

The liquid guide 212 is disposed upstream of the airflow path 40 and the flavor source 311 is disposed downstream thereof.

Air flowing in through the air inlet hole 21 in response to inhalation by the user is mixed with the aerosol generated by the heater 211. A gas obtained as a result of mixture passes through the flavor source 311 and is conveyed to the air outlet hole 42 as indicated by the arrow 41. To the gas obtained by mixing the aerosol and air, the flavor component of the flavor source 311 is imparted when the gas passes through the flavor source 311.

Note that the aerosol generation device 1 may be used without the flavor source 311 being mounted in the cartridge 30.

The inhalation port 31 is a member that is held in the user's mouth during inhalation. The inhalation port 31 is provided with the air outlet hole 42. When the user holds the inhalation port 31 in their mouth and inhales, the user can take the gas, which is a mixture of the aerosol and air, into their oral cavity.

Although an example internal configuration of the aerosol generation device 1 has been described above, the configuration illustrated in Fig. 2 is only one form.

For example, the aerosol generation device 1 can be configured so as not to include the cartridge 30. In this case, the cartridge 20 is provided with the inhalation port 31.

The aerosol generation device 1 can include a plurality of types of aerosol sources. A plurality of types of aerosols generated from the plurality of types of aerosol sources may be mixed inside the airflow path 40 and may be chemically react to thereby generate another type of aerosol.

A method for atomizing the aerosol source is not limited to heating by the heater 211. For example, an induction heating technique may be used to atomize the aerosol source.

### <Control of Length of Real Heating Time>

### <Assumptions about Control>

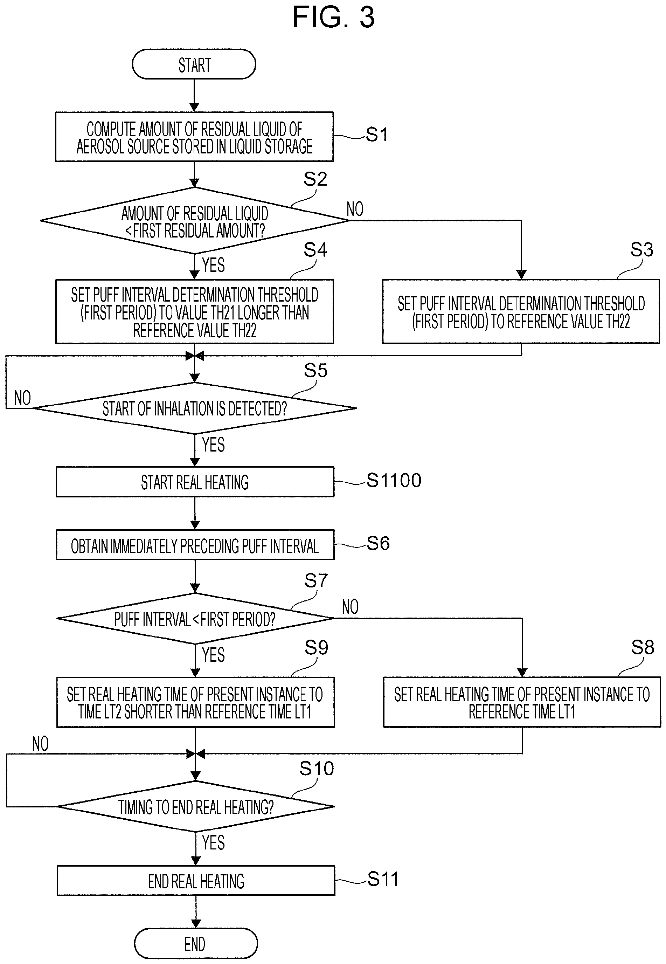

Fig. 3 is a flowchart for explaining example control of the real heating time by the controller 117 (see Fig. 2) used in Embodiment 1. The control by the controller 117 is implemented through execution of a program. Therefore, the controller 117 is one form of a computer. In Fig. 3, the symbol S is used to represent step.

In this embodiment, "real heating time" is used to mean the time in which the aerosol source held in the liquid guide 212 (see Fig. 2) is heated and atomized and an aerosol is generated. A temperature at which the aerosol source is heated in the real heating time is an example of a first temperature.

In the flowchart illustrated in Fig. 3, prior to control of the length of the real heating time, a determination threshold for the length of a puff interval is set.

As described above, the aerosol source is supplied to the liquid guide 212 by a capillary action. In this embodiment, a control method in a case where the speed of liquid sending by the capillary action depends on the amount of residual liquid will be described. For example, a control method when, in a situation in which the liquid supply speed decreases due to a decrease in the amount of residual liquid, the liquid amount of the aerosol source that can be supplied during a single instance of inhalation becomes smaller than in a case where the amount of residual liquid is large will be described. When the real heating time is the same regardless of the amount of residual liquid, there is a possibility that supply of the aerosol source is not in time and an event similar to drying up occurs.

In this embodiment, a method for controlling the length of the real heating time by taking into consideration the amount of residual liquid will be described.

Note that in this embodiment, supply of electric power to the heater 211 coincides with inhalation by the user using the aerosol generation device 1 (see Fig. 1). Inhalation by the user using the aerosol generation device 1 is hereinafter also referred to as "inhalation of an aerosol" generated from the aerosol source.

The temperature of the heater 211 rises in response to the start of supply of electric power and drops in response to the stop of supply of electric power. In this embodiment, the temperature of the heater 211 rises to the boiling point of the aerosol or above in response to the start of supply of electric power and drops to the boiling point of the aerosol or below in response to the stop of supply of electric power. However, in this embodiment, the time in which electric power is supplied to the heater 211 and the time in which the aerosol is generated from the liquid guide 212 are considered to be substantially the same.

Strictly speaking, electric power supplied immediately after the start of supply is consumed more for a rise in the temperature of the aerosol source than for vaporization of the aerosol source.

Therefore, a time lag is present between the start of supply of electric power and the start of aerosol generation.

However, this time difference is very small, and therefore, a description will be given in this embodiment without taking into consideration the time difference.

### <Details of Control>

The controller 117 in this embodiment first computes the amount of residual liquid (that is, the residual amount) of the aerosol source stored in the liquid storage 213 (see Fig. 2) (step 1).

The controller 117 in this embodiment computes the amount of residual liquid at the start time point of each instance of inhalation by integrating the amount of consumption of the aerosol source in every instance of inhalation and subtracting the computed value of integral from the initial value.

Note that the amount of consumption of the aerosol source in every instance of inhalation can be computed as a function of the amount of electric power supplied to the heater 211.

After computing the amount of residual liquid, the controller 117 determines whether the amount of residual liquid is smaller than a first residual amount (step 2). The first residual amount is set in advance. The first residual amount is determined on the basis of, for example, a relationship between the speed of liquid sending in accordance with the amount of residual liquid and a liquid amount necessary when the real heating time is a reference time LT1.

If the amount of residual liquid is larger than or equal to the first residual amount, the controller 117 obtains a negative result in step 2. In this case, the controller 117 sets a puff interval determination threshold (first period) to a reference value TH22 (step 3).

On the other hand, if the amount of residual liquid is smaller than the first residual amount, the controller 117 obtains a positive result in step 2. In this case, the controller 117 sets the puff interval determination threshold (first period) to a value TH21 longer than the reference value TH22 (step 4).

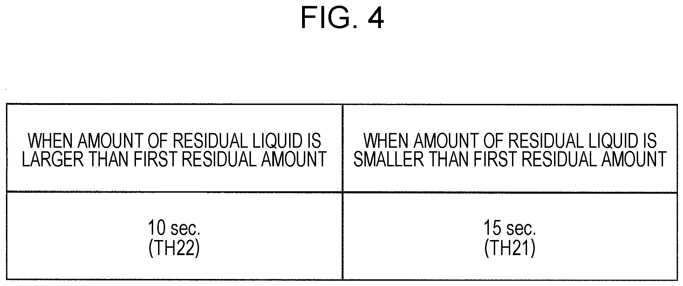

Fig. 4 is a diagram for explaining example settings of the determination threshold (first period) in accordance with the amount of residual liquid. In Fig. 4, the determination threshold (that is, the value TH22) when the amount of residual liquid is larger than the first residual amount is 10 seconds, and the determination threshold (that is, the value TH21) when the amount of residual liquid is smaller than the first residual amount is 15 seconds. As the determination threshold is longer, the same puff interval is more likely to be determined to be a short puff interval.

Note that the numerical values of the determination threshold indicated in Fig. 4 are not definite values and differ depending on, for example, the adopted heating mode as described in another embodiment described below.

Consequently, setting of the puff interval determination threshold ends.

Referring back to Fig. 3, a description will be given.

After the end of setting the puff interval determination threshold, the controller 117 determines whether the start of inhalation is detected by the puff sensor 112 (step 5).

If the start of inhalation of an aerosol by the user is not detected, the controller 117 obtains a negative result in step 5. As long as a negative result is obtained in step 5, the controller 117 repeats the determination in step 5.

On the other hand, if the start of inhalation of an aerosol by the user is detected, the controller 117 obtains a positive result in step 5. If a positive result is obtained in step 5, the controller 117 starts real heating (step 1100), and thereafter, obtains the immediately preceding puff interval (step 6).

In this embodiment, the immediately preceding puff interval is given as the time from the end of the immediately preceding instance of inhalation (puff) to the start of the present instance of inhalation (puff). The puff interval may be measured by, for example, a timer or may be calculated as the difference between the end time of the immediately preceding instance of inhalation and the start time of the present instance of inhalation. The times are obtained from, for example, a timer incorporated in the controller 117 or an integrated circuit or the like that implements a timer function.

After obtaining the puff interval, the controller 117 determines whether the puff interval is shorter than the first period (step 7).

If the puff interval is longer than or equal to the first period, the controller 117 obtains a negative result in step 7. In this case, the controller 117 sets the real heating time of the present instance to the reference time LT1 (step 8). In this embodiment, as the reference time LT1, for example, 2.4 seconds is used. As a matter of course, this value is an example of the reference time. The reference time LT1 is set to a time in which drying up caused by inhalation of the aerosol by an assumed typical user does not occur when the puff interval is longer than the first period.

On the other hand, if the puff interval is shorter than the first period, the controller 117 obtains a positive result in step 7. This case is referred to as "short puff'.

When a short puff is detected, the controller 117 sets the real heating time of the present instance to a time LT2 shorter than the reference time LT1 (step 9). In this embodiment, only the real heating time is shortened, and the voltage value and the current value of a voltage and a current supplied to the heater 211 remain the same regardless of a difference in the puff interval.

In this embodiment, as the time LT2, for example, 1.7 seconds is used. As a matter of course, this value is an example of the real heating time for a short puff. As the time LT2 is shorter, a drying up event in which an aerosol is not generated even when the aerosol source is heated is less likely to occur.

After setting the real heating time in step 8 or step 9, the controller 117 determines whether the timing to end the real heating comes (step 10).

In this embodiment, the real heating ends in response to, for example, the expiration of the set real heating time, the end of inhalation of the aerosol by the user, or an operation for forced termination. Therefore, even when the set real heating time does not yet expire, supply of electric power to the heater 211 ends if the real heating is determined to be ended. The passage of the real heating time is monitored on the basis of the time elapsed since the start of supply of electric power to the heater 211.

Note that examples of the operation for forced termination include pressing and holding down of the power supply button 11 (see Fig. 1). Pressing and holding down of the power supply button 11 refers to the power supply button 11 being kept pressed for a predetermined time or longer. When, the power supply button 11 is kept pressed for, for example, 3 seconds or longer, the controller 117 determines that a pressing and holding down operation is performed.

As long as a negative result is obtained in step 10, the controller 117 repeats the determination in step 10. During this period, supply of electric power to the heater 211 continues.

On the other hand, if a positive result is obtained in step 10, the controller 117 ends the real heating (step 11). That is, the controller 117 stops supply of electric power to the heater 211.

Consequently, one cycle of inhalation ends.

Note that in a case of a short puff, the real heating time is shorter than the reference time, and therefore, the amount of electric power supplied to the heater 211 during one cycle of inhalation is smaller than the amount of electric power supplied in a case of the reference time.

Figs. 5A to 5C are diagrams for explaining a relationship between a puff interval and a setting of the real heating time in Embodiment 1. Fig. 5A illustrates example timings of inhalation (puffs), Fig. 5B illustrates example settings of the real heating time when the amount of residual liquid is larger than a first threshold, and Fig. 5C illustrates example settings of the real heating time when the amount of residual liquid is smaller than the first threshold. The vertical axis in Fig. 5A represents the puff intensity, the vertical axis in Figs. 5B and 5C represents the intensity of heating, and the horizontal axis in Figs. 5A to 5C represents time. The puff intensity is detected by the puff sensor. Although the puff intensity is detected as the presence or absence of a puff in this embodiment, the puff intensity may be defined as the amount of inhaled air. The intensity of heating is the amount of electric power and is given as the product of the voltage value and the current value of a voltage and a current supplied to the heater 211.

In Fig. 5A, the number of instances of inhalation (puff) is five.

In Fig. 5A, the interval between the first puff and the second puff is IT 1, the interval between the second puff and the third puff is IT2, the interval between the third puff and the fourth puff is IT3, and the interval between the fourth puff and the fifth puff is IT4.

The first puff interval IT1 is longer than the first period (that is, the reference value TH21) that is for the case where the amount of residual liquid is small.

The second puff interval IT2 is shorter than the first period (that is, the reference value TH21) that is for the case where the amount of residual liquid is small, and longer than the first period (that is, the reference value TH22) that is for the case where the amount of residual liquid is large.

The third and fourth puff intervals IT3 and IT4 are shorter than the first period (that is, the reference value TH22) that is for the case where the amount of residual liquid is large.

For example, when the amount of residual liquid is larger than the first residual amount, the first period is set to the reference value TH22.

Accordingly, the first puff intervals IT1 and IT2 are determined to be longer than the first period, and the third and fourth puff intervals IT3 and IT4 are determined to be shorter than the first period. That is, the third and fourth puff intervals are determined to be short puffs.

As a result, as illustrated in Fig. 5B, the real heating time corresponding to the first to third puffs is set to the reference time LT1, and the real heating time corresponding to the fourth and fifth puffs is set to the time LT2 shorter than the reference time LT1.

For the fourth and fifth puffs determined to be short puffs, the real heating time is shortened, and therefore, even when the amount of aerosol source supplied to the heater 211 before the start of inhalation is small, drying up does not occur during the fourth and fifth puffs.

On the other hand, when the amount of residual liquid is smaller than the first residual amount, the first period is set to the reference value TH21 longer than the reference value TH22. As described above, the first puff interval IT1 is longer than the first period (that is, the reference value TH21) that is for the case where the amount of residual liquid is small while all of the second puff interval IT2, the third puff interval IT3, and the fourth puff interval IT4 are shorter than the first period (that is, the reference value TH21) that is for the case where the amount of residual liquid is small.

Accordingly, as illustrated in Fig. 5C, the real heating time corresponding to the first and second puffs is set to the reference time LT1 while the real heating time corresponding to the third, fourth, and fifth puffs is set to the time LT2 shorter than the reference time LT1.

In this embodiment, in the case where the amount of residual liquid is small, the supplied amount of the aerosol source becomes smaller than in the case where the amount of residual liquid is large. Accordingly, in Fig. 5C, the third puff for which an insufficient supplied amount is no concern if the amount of residual liquid is large is determined to be a short puff, and the real heating time is made shorter than the reference time LT1.

As a result, the time IT11, immediately before the fourth puff, in which supply of electric power to the heater 211 stops becomes longer than the third puff interval IT3. Accordingly, the supplied amount of the aerosol source guided to the heating region increases. As a result, even for the third puff, drying up does not occur during the puff, as well as for the fourth and fifth puffs.

Although the period of inhalation of the aerosol by the user and the heating time of the heater 211 are made to coincide with each other within the real heating time set in advance in Figs. 5A to 5C, the real heating may be started in response to an ON operation on the power supply button 11 or the real heating may be continued until the real heating time expires even after the end of inhalation by the user.

In these cases, although the puff interval does not coincide with the time in which the real heating stops, drying up at the time of a short puff in the case where the amount of residual liquid is small can be effectively suppressed as in the above-described example control.

### <Embodiment 2>

In Embodiment 2, the amount of residual liquid is obtained as a measured value. Note that the external configuration of the aerosol generation device 1 in this embodiment is the same as in Embodiment 1.

Fig. 6 is a diagram schematically illustrating an internal configuration of the aerosol generation device 1 assumed in Embodiment 2. In Fig. 6, a part corresponding to a corresponding part in Fig. 2 is assigned a corresponding reference sign and illustrated.

The aerosol generation device 1 in this embodiment is provided with an amount-of-residual-liquid sensor 113A. The amount-of-residual-liquid sensor 113A is an example of the first sensor.

The amount-of-residual-liquid sensor 113A is a sensor that detects the amount of residual liquid of the aerosol source stored in the liquid storage 213 and is, for example, a level switch, a level meter, a capacitive sensor, or a sensor that measures the distance to the liquid surface. The distance to the liquid surface can be measured as, for example, the time taken for an ultrasonic wave, an electromagnetic wave, or a laser to return after reflected on the liquid surface.

Note that the amount of residual liquid that is definitively used is corrected by the controller 117 using information about the orientation of the aerosol generation device 1. As the information about the orientation, for example, an output signal of a gyro sensor is used.

Fig. 7 is a flowchart for explaining example control of the real heating time by the controller 117 (see Fig. 2) used in Embodiment 2. In Fig. 7, a part corresponding to a corresponding part in Fig. 3 is assigned a corresponding reference sign and illustrated.

In this embodiment, the controller 117 obtains the amount of residual liquid of the aerosol source stored in the liquid storage 213 (see Fig. 2) from the amount-of-residual-liquid sensor 113A (step 21). Note that the level of the liquid surface of the aerosol source stored in the liquid storage 213 differs depending on the orientation of the aerosol generation device 1 even when the amount of residual liquid is the same. Accordingly, the controller 117 corrects the measured amount of residual liquid by using information about the orientation of the aerosol generation device 1 and uses the corrected amount of residual liquid in the determination in step 2.

Note that the details of processes in step 2 and the subsequent steps are the same as in Embodiment 1.

### <Embodiment 3>

In Embodiment 3, control of making the puff interval determination threshold longer in a step-by-step manner in the case where the amount of residual liquid is small and where a short puff determination is consecutively made a plurality of times will be described.

Note that the other configurations of the aerosol generation device 1 (see Fig. 1) in this embodiment are the same as in Embodiment 1. That is, the external configuration and the internal configuration of the aerosol generation device 1 are the same as in Embodiment 1.

Fig. 8 is a flowchart for explaining example control of the real heating time by the controller 117 (see Fig. 2) used in Embodiment 3. In Fig. 8, a part corresponding to a corresponding part in Fig. 3 is assigned a corresponding reference sign and illustrated. The control by the controller 117 is implemented through execution of a program.

First, the controller 117 computes the amount of residual liquid (that is, the residual amount) of the aerosol source stored in the liquid storage 213 (see Fig. 2) (step 1).

Although the amount of residual liquid is computed by calculation in this embodiment, the amount of residual liquid can be obtained by using the amount-of-residual-liquid sensor 113A as in Embodiment 2.

Next, the controller 117 determines whether the amount of residual liquid is smaller than the first residual amount (step 2).

If a negative result is obtained in step 2, the controller 117 sets the puff interval determination threshold (first period) to the reference value TH22 (step 3).

On the other hand, if a positive result is obtained in step 2, the controller 117 obtains records of the puff intervals of a plurality of past instances including the puff interval of the present instance (step 31).

The number of puff interval records to be obtained is set in advance. For example, records for three to five instances are obtained. The number of puff interval records to be obtained is set to a number with which the latest inhalation tendency can be detected.

After obtaining records of the puff intervals of a plurality of past instances, the controller 117 obtains the number of times a puff interval shorter than the first period consecutively appears up to the present instance (step 32). As the number of consecutive times is greater, the possibility that the liquid temperature of the aerosol source at the start of inhalation is high becomes higher. As the liquid temperature is high, the liquid sending speed also becomes high, however, there is a limit to liquid sending by a capillary action, and the possibility that supply of the aerosol source becomes not in time becomes high in the latter part of the real heating period.

In this embodiment, as the first period used in step 32, a value set at the time point of processing is used. Therefore, when the first period is set to a value longer than the reference value TH22 in step 4 or in step 34 performed at the time of inhalation in the immediately preceding instance, the value is used.

However, the first period used in step 32 may be fixed to the reference value TH22.

Alternatively, in step 32, the maximum number of consecutive times within the obtained records may be obtained instead of the number of consecutive times up to the present instance. Without the number of consecutive times up to the present instance, the possibility that the liquid temperature becomes high can be known.

Subsequently, the controller 117 determines whether the number of consecutive times is greater than a first number of times (step 33).

If the number of consecutive times is less than or equal to the first number of times, the controller 117 obtains a negative result in step 33. In this case, the controller 117 sets the puff interval determination threshold (first period) to the reference value TH21 longer than the reference value TH22 (step 4).

On the other hand, if the number of consecutive times is greater than the first number of times, the controller 117 obtains a positive result in step 33. In this case, the controller 117 sets the puff interval determination threshold (first period) to a longer reference value TH23 in a step-by-step manner as the number of times a short puff interval consecutively appears is greater (step 34).

In a case where a short puff consecutively appears, drying up is more likely to occur than in a case where a short puff does not consecutively appear, and therefore, the reference value TH23 is set to a value longer than the reference value TH21. This is because as the determination threshold is longer, the number of instances of inhalation that is determined to be a short puff increases, the real heating time is shortened, and consequently, drying up is less likely to occur.

In this embodiment, the controller 117 sets the determination threshold (first period) to the longer reference value TH23 in a step-by-step manner as the number of consecutive times is greater. For example, the determination threshold is made longer by a value equal to 1 second × the number of consecutive times. In this embodiment, the reference value TH23 is set to a time longer than the reference value TH21. Accordingly, the reference value TH23 is set with reference to the reference value TH21. However, the reference value TH23 may be calculated with reference to a reference value longer than the reference value TH21.

In this embodiment, the determination threshold is increased linearly in accordance with the number of consecutive times. However, the determination threshold may be increased nonlinearly in accordance with, for example, a quadric curve.

After setting the determination threshold (first period) in step 3, step 4, or step 34, the controller 117 performs step 5 to step 11 in sequence and ends one cycle of inhalation.

In this embodiment, as the number of times a short puff consecutively appears is greater, the controller 117 makes the determination threshold (first period) longer so that the same puff interval is more likely to be determined to be a short puff.

This is because as the number of times a short puff consecutively appears (hereinafter referred to as "number of consecutive short puffs) in a state in which the amount of residual liquid is small increases, the possibility that supply of the aerosol source to the heater 211 becomes not in time becomes higher. That is, drying up is more likely to occur.

However, in this embodiment, as the number of consecutive short puffs increases, the real heating time becomes shorter, and therefore, drying up is effectively suppressed.

Fig. 9A is a diagram for explaining example settings of the puff interval determination threshold (first period) in Embodiment 3. The example illustrated in Fig. 9A assumes a case where the first number of times is two.

First, when the amount of residual liquid is larger than the first residual amount, the speed of liquid sending from the aerosol source has no problem, and therefore, the determination threshold is set to 10 seconds, which is the reference value TH22.

On the other hand, when the amount of residual liquid is smaller than the first residual amount, the length of the determination threshold (first period) differs in accordance with the number of consecutive short puffs.

In this embodiment, even when the puff interval is short, the determination threshold is set to a fixed value (that is, TH21) longer than the reference value as long as the number of consecutive short puffs is two or less. Specifically, the determination threshold is set to 15 seconds.

When the number of consecutive short puffs is three or more, the determination threshold is made longer in increments of 1 second. Specifically, the determination threshold is made longer to 16 seconds, 17 seconds, and so on.

For example, when the number of consecutive short puffs is five, even when the puff interval is 17 seconds, the real heating time is set to the time LT2 shorter than the reference time LT1.

When this control is adopted, the possibility that the time from the end of the real heating time to the next inhalation (puff) becomes long becomes higher than in a case where a fixed value is used as the puff interval determination threshold (first period), and the liquid amount of the aerosol source that is sent before the next inhalation (puff) can be increased. When the time before the next inhalation (puff) becomes long, the liquid temperature of the aerosol source accordingly decreases, and drying up is less likely to occur.

Although the amount of residual liquid and the first residual amount are compared with each other, and thereafter (that is, after step 1 and step 2), the length of the determination threshold (first period) is set in accordance with the number of consecutive instances of inhalation whose puff interval is short in this embodiment, the number of consecutive instances of inhalation whose puff interval is short and the first number of times may be compared with each other, and thereafter (that is, after steps 31 to 33), the amount of residual liquid and the first residual amount may be compared with each other, and the length of the puff interval determination threshold (first period) may be set in accordance with the result of comparison.

Fig. 9B is a flowchart for explaining other example control in Embodiment 3. In Fig. 9B, a part corresponding to a corresponding part in Fig. 8 is assigned a corresponding reference sign and illustrated.

In Fig. 9B, step 31, step 32, and step 33 are performed in sequence.

If a negative result is obtained in step 33 (that is, the number of consecutive times is less than or equal to the first number of times), the controller 117 sets the puff interval determination threshold (first period) to the reference value TH22 (step 3).

On the other hand, if a positive result is obtained in step 33 (that is, the number of consecutive times is greater than the first number of times), the controller 117 computes the amount of residual liquid (that is, the residual amount) of the aerosol source stored in the liquid storage 213 (see Fig. 2) (step 1), and subsequently, determines whether the amount of residual liquid is smaller than the first residual amount (step 2).

In Fig. 9B, if a positive result is obtained in step 2 (that is, the amount of residual liquid is smaller than the first residual amount), the controller 117 sets the puff interval determination threshold (first period) to the longer reference value TH23 in a step-by-step manner as the number of times a short puff interval consecutively appears is greater (step 34).

On the other hand, if a negative result is obtained in step 2 (that is, the amount of residual liquid is larger than the first residual amount), the controller 117 sets the puff interval determination threshold (first period) to the reference value TH21 longer than the reference value TH22 (step 4).

After setting the determination threshold (first period) in step 3, step 4, or step 34, the controller 117 performs step 5 to step 11 in sequence and ends one cycle of inhalation.

### <Embodiment 4>

In this embodiment, control of variably setting the puff interval determination threshold (first period) in accordance with the amount of residual liquid of the aerosol source instead of setting the puff interval determination threshold (first period) to a fixed value that is determined on a per condition basis will be described. That is, in this embodiment, as the puff interval determination threshold, the reference value TH21 and a variable value are used instead of using any of the reference value TH21 and the reference value TH22.

Note that the other configurations of the aerosol generation device 1 (see Fig. 1) in this embodiment are the same as in Embodiment 1. That is, the external configuration and the internal configuration of the aerosol generation device 1 are the same as in Embodiment 1.

Fig. 10 is a flowchart for explaining example control of the real heating time by the controller 117 (see Fig. 2) used in Embodiment 4. In Fig. 10, a part corresponding to a corresponding part in Fig. 3 is assigned a corresponding reference sign and illustrated. The control by the controller 117 is implemented through execution of a program.

In this embodiment as well, the controller 117 computes the amount of residual liquid of the aerosol source stored in the liquid storage 213 (see Fig. 2) (step 1).

After computing the amount of residual liquid, the controller 117 determines whether the amount of residual liquid is smaller than the first residual amount (step 2).

Although the amount of residual liquid is computed by calculation in this embodiment, the amount of residual liquid can be obtained by using the amount-of-residual-liquid sensor 113A as in Embodiment 2.

If a negative result is obtained in step 2, the controller 117 sets the puff interval determination threshold (first period) to the reference value TH22 (step 3).

On the other hand, if a positive result is obtained in step 2, the controller 117 sets the puff interval determination threshold (first period) to a reference value TH24 longer than the puff interval of the immediately preceding instance (step 41). Note that the reference value TH24 is a value longer than the reference value TH22. This is because the amount of residual liquid may be small, and therefore, there is a possibility of drying up when the reference value TH24 is shorter than the reference value TH22.

In this embodiment, when, for example, the puff interval of the immediately preceding instance is 11 seconds, the reference value TH24 is set to 12 seconds. Similarly, when the puff interval of the immediately preceding instance is 15 seconds, the reference value TH24 is set to 16 seconds. That is, in this embodiment, a value calculated by adding 1 second to the puff interval of the immediately preceding instance is used as the reference value TH24. Note that this way of giving the reference value TH24 is an example.

As a result, when the puff interval of the present instance has a length the same as that of the immediately preceding instance, a short puff determination is made with certainty.

When a short puff determination is made, the real heating time is set to the time LT2 shorter than the reference time LT1. Accordingly, the period in which supply of electric power to the heater 211 is stopped becomes longer than the actual puff interval with certainty. That is, this leads to an increase in the supplied amount of the aerosol source guided to the heating region, and the occurrence of drying up can be effectively suppressed.

Note that the reference value TH24 need not be obtained as a value obtained by adding a fixed value (for example, 1 second) to the puff interval of the immediately preceding instance as described above and may be computed by using a predetermined function. This function may be a quadratic function or a cubic function, may be an exponential function or a logarithmic function, or may be another function.

The reference value TH24, which is set in accordance with the puff interval of the immediately preceding instance, may be read from a prepared table and used.

Even for the same puff interval, the reference value TH24 may be made variable in accordance with the amount of residual liquid. For example, as the amount of residual liquid is smaller, the reference value TH24 may be set to a larger value even for the same pass interval. That is, as the amount of residual liquid is smaller, a short puff determination may be more likely to be made in step 7 (see Fig. 3).

### <Embodiment 5>

This embodiment assumes a case of providing a function of preliminarily heating the heater 211 (see Fig. 2) prior to real heating.

Note that the other configurations of the aerosol generation device 1 (see Fig. 1) in this embodiment are the same as in Embodiment 1. That is, the external configuration and the internal configuration of the aerosol generation device 1 are the same as in Embodiment 1.

Figs. 11A and 11B are diagrams for explaining a preliminary heating time. Fig. 11A illustrates a relationship between the positions of the preliminary heating time and the real heating time and Fig. 11B illustrates changes in the temperature of the aerosol source. The vertical axis in Fig. 11A represents the intensity of heating, the vertical axis in Fig. 11B represents the temperature, and the horizontal axis in Figs. 11A and 11B represents time.

The preliminary heating time is the time for preliminary heating and is positioned immediately before the real heating time.

Preliminary heating is provided for heating in advance the liquid temperature of the aerosol source in the liquid guide 212 (see Fig. 2) to the room temperature or above and below the boiling point. Preliminary heating is a technique for reducing the time lag between the start of supply of electric power to the heater 211 and generation of an aerosol. A temperature at which the aerosol source is heated in the preliminary heating time is an example of a second temperature.

With preliminary heating, the liquid temperature of the aerosol source can be increased in advance. Accordingly, electric power supplied in the real heating time can be allotted more to generation of an aerosol than to a rise in the liquid temperature of the aerosol source. As a result, an aerosol can be generated immediately after the start of the real heating time, and consequently, the total amount of aerosol generated in the real heating time can be increased.

The time from the start of the real heating time to the time when the temperature of the aerosol source reaches the boiling point is TD 1 when preliminary heating is not used, but can be reduced to TD2 (< TD1) when preliminary heating is used. Accordingly, when the length of the real heating time is the same as in the case of not using preliminary heating, more aerosol can be generated in the case of using the preliminary heating.

Note that in Figs. 11(A) and (B), the real heating time LT11 in the case of using preliminary heating is made shorter than the real heating time LT1 in the case of not using preliminary heating. This is for making the total amount of aerosol generated in the real heating time be the same.

In other words, when the amount of generated aerosol is controlled so as to be the same as in the case without preliminary heating, the real heating time LT11 in the case of using preliminary heating can be made shorter than the real heating time LT1 in the case without preliminary heating.

One reason why generation of an aerosol is accelerated by preliminary heating is that the viscosity of the aerosol source at the start of the real heating time becomes lower than in the case of not using preliminary heating. This is because as the viscosity of the aerosol source is lower, the speed of liquid sending to the liquid guide 212 increases, and consequently, the amount of supplied liquid increases.

As the preliminary heating time is longer, the amount of consumed electric power accordingly increases. Accordingly, the length of the preliminary heating time needs to be set by taking into consideration balance against the amount of electric power consumed in the real heating time.

Fig. 12 is a diagram for explaining example settings of the determination threshold in accordance with whether preliminary heating is used and the amount of residual liquid. (A) illustrates the case without preliminary heating and (B) illustrates the case with preliminary heating. Here, "without preliminary heating" and "with preliminary heating" do not mean whether or not the function of preliminary heating is provided but mean whether or not the function of preliminary heating is used.

The example settings of the determination threshold illustrated in Fig. 12(A) are the same as in Embodiment 1. That is, in the case "without preliminary heating", the determination threshold is set to 10 seconds when the amount of residual liquid is larger than the first threshold, and the determination threshold is set to 15 seconds when the amount of residual liquid is smaller than the first threshold.

On the other hand, as illustrated in Fig. 12(B), in the case "with preliminary heating", the determination threshold is set to 10 seconds when the amount of residual liquid is larger than the first threshold, and the determination threshold is set to 12 seconds when the amount of residual liquid is smaller than the first threshold.

In Fig. 12, the value of the determination threshold when the amount of residual liquid is larger than the first threshold is 10 seconds both in the case "without preliminary heating" and the case "with preliminary heating". The reason is that when the amount of residual liquid is large, a shortage does not occur in supply of the aerosol source regardless of whether preliminary heating is used.

The value of the determination threshold when the amount of residual liquid is smaller than the first threshold is 15 seconds in the case "without preliminary heating" while 12 seconds in the case "with preliminary heating". This is because in the case of using preliminary heating, the supplied amount of the aerosol source is larger than in the case of not using preliminary heating even when the amount of residual liquid is small, and drying up is less likely to occur.

Note that all of the numbers of seconds illustrated in Fig. 12 are examples and are not limited to the indicated values.

Fig. 13 is a flowchart for explaining example control of the real heating time by the controller 117 (see Fig. 2) used in Embodiment 5. In Fig. 13, a part corresponding to a corresponding part in Fig. 3 is assigned a corresponding reference sign and illustrated.

The controller 117 in this embodiment first determines whether preliminary heating is used (step 51). That is, the controller 117 determines whether a preliminary heating mode is ON or the preliminary heating mode is OFF.

In other words, the aerosol generation device 1 in this embodiment has the preliminary heating mode, and whether to use the aerosol generation device 1 in a state in which the preliminary heating mode is ON or in a state in which the preliminary heating mode is OFF depends on the user's choice. For example, the preliminary heating mode can be turned ON or OFF by a specific operation on the power supply button 11 (see Fig. 1) or in response to an instruction from an external device, such as a smartphone, connected by Bluetooth (registered trademark) or USB (Universal Serial Bus).

The aerosol generation device 1 may be provided with a dedicated button for turning ON or OFF the preliminary heating mode.

If a negative result is obtained in step 51, the controller 117 performs operations similar to those in, for example, Embodiment 1. That is, if the preliminary heating mode is OFF, the controller 117 sets the puff interval determination threshold and the real heating time in accordance with the flowchart illustrated in Fig. 3.

Specifically, the controller 117 computes the amount of residual liquid of the aerosol source stored in the liquid storage 213 (see Fig. 2) (step 1), obtains a negative result in step 2 if the amount of residual liquid is larger than the first residual amount and performs step 3, or obtains a positive result in step 2 if the amount of residual liquid is smaller than the first residual amount and performs step 4. Thereafter, the controller 117 performs step 5 to step 11 in sequence and ends one cycle of inhalation.

Although the amount of residual liquid is computed by calculation in this embodiment, the amount of residual liquid can be obtained by using the amount-of-residual-liquid sensor 113A as in Embodiment 2.

On the other hand, if a positive result is obtained in step 51, the controller 117 performs the following process. That is, when the preliminary heating mode is ON, the controller 117 computes the amount of residual liquid of the aerosol source stored in the liquid storage 213 (step 1A), obtains a negative result in step 2A if the amount of residual liquid is larger than the first residual amount, and performs step 3. The process so far is the same as in the case without preliminary heating. Note that the threshold used in determination in step 2A may be different from that used in step 2. For example, the threshold used in determination in step 2A may be smaller than the threshold used in determination in step 2. The determination threshold (first period) in the case where a negative result is obtained in step 2A may be shorter than the determination threshold (first period) in the case where a negative result is obtained in step 2.

When a positive result is obtained in step 2A after a positive result is obtained in step 51, the controller 117 sets the puff interval determination threshold (first period) to the reference value TH23 longer than the reference value TH22 (step 52).

The reference value TH23 here is a value shorter than the reference value TH21 set in step 4. Here, the reference value TH21 corresponds to 15 seconds in Fig. 12, the reference value TH22 corresponds to 10 seconds in Fig. 12, and the reference value TH23 corresponds to 12 seconds in Fig. 12.

After setting the determination threshold (first period) in step 3, step 4, or step 52, the controller 117 performs steps 5 to 11 in sequence and ends one cycle of inhalation.

In the case where the preliminary heating mode is ON, as long as the amount of residual liquid is the same, the capability of sending liquid of the aerosol source is higher than in the case where the preliminary heating mode is OFF. Therefore, even when the amount of residual liquid is small, the number of times control of shortening the real heating time is performed can be decreased.

### <Embodiment 6>

In this embodiment, a case will be described where even in the case of using preliminary heating, when a short puff determination is consecutively made in the state in which the amount of residual liquid is small, the puff interval determination threshold (first period) is made longer in a step-by-step manner as the number of consecutive times is greater so that the real heating time is more likely to be shortened.

In other words, this embodiment corresponds to an example combination of Embodiment 3 and Embodiment 5.

Note that the other configurations of the aerosol generation device 1 (see Fig. 1) in this embodiment are the same as in Embodiment 1. That is, the external configuration and the internal configuration of the aerosol generation device 1 are the same as in Embodiment 1.

Fig. 14A is a flowchart for explaining example control of the real heating time by the controller 117 (see Fig. 2) used in Embodiment 6. In Fig. 14A, a part corresponding to a corresponding part in Fig. 3, Fig. 8, and Fig. 13 is assigned a corresponding reference sign and illustrated. The control by the controller 117 is implemented through execution of a program.

The controller 117 in this embodiment first determines as well, whether preliminary heating is used (step 51).

If a negative result is obtained in step 51, the controller 117 performs operations similar to those in, for example, Embodiment 1.

That is, the controller 117 sets the puff interval determination threshold and the real heating time in accordance with the flowchart illustrated in Fig. 3.

Specifically, the controller 117 computes the amount of residual liquid of the aerosol source stored in the liquid storage 213 (see Fig. 2) (step 1), obtains a negative result in step 2 if the amount of residual liquid is larger than the first residual amount and performs step 3, or obtains a positive result in step 2 if the amount of residual liquid is smaller than the first residual amount and performs step 4. Thereafter, the controller 117 performs step 5 to step 11 in sequence and ends one cycle of inhalation.

Although the amount of residual liquid is computed by calculation in this embodiment, the amount of residual liquid can be obtained by using the amount-of-residual-liquid sensor 113A as in Embodiment 2.

On the other hand, if a positive result is obtained in step 51, the controller 117 performs the following process.

First, the controller 117 computes the amount of residual liquid of the aerosol source stored in the liquid storage 213 (step 1A), and if the amount of residual liquid is larger than the first residual amount, obtains a negative result in step 2A. In this case, the flow proceeds to step 3, and the controller 117 sets the determination threshold (first period) to the reference value TH22.

On the other hand, if a positive result is obtained in step 2A after step 51 in which a positive result is obtained, the controller 117 obtains records of the puff intervals of a plurality of past instances including the puff interval of the present instance (step 31).

After obtaining records of the puff intervals of a plurality of past instances, the controller 117 obtains the number of times a puff interval shorter than the first period consecutively appears up to the present instance (step 32).

Subsequently, the controller 117 determines whether the number of consecutive times is greater than the first number of times (step 33).

If the number of consecutive times is less than or equal to the first number of times, the controller 117 obtains a negative result in step 33. In this case, the controller 117 sets the puff interval determination threshold (first period) to the reference value TH23 longer than the reference value TH22 (step 61). Note that the reference value TH23 here is a value shorter than the reference value TH21 that is used when the amount of residual liquid is small in the case without preliminary heating. The reference value TH21 and the reference value TH23 here correspond to the relationship illustrated in Fig. 12.

On the other hand, if the number of consecutive times is greater than the first number of times, the controller 117 obtains a positive result in step 33. In this case, the controller 117 sets the puff interval determination threshold (first period) to the longer reference value TH24 in a step-by-step manner as the number of times a short puff interval consecutively appears is greater (step 62).

This is because even in the case of using preliminary heating, in the case where a short puff consecutively appears in the state in which the amount of residual liquid is small, drying up is more likely to occur than in the case where a short puff does not consecutively appear. The reference value TH24 here is set to a base value longer than the reference value TH23. The initial value of the reference value TH24 may be shorter than the reference value TH21 as long as the initial value is a value longer than the reference value TH23. However, as the number of times a short puff consecutively appears increases, the reference value TH24 becomes longer than the reference value TH21.

After setting the determination threshold (first period) in step 3, step 4, step 61, or step 62, the controller 117 performs step 5 to step 11 and step 1100 in sequence and ends one cycle of inhalation.

In this embodiment as well, as described in Embodiment 3, the number of consecutive instances of inhalation whose puff interval is short and the first number of times may be compared with each other, and thereafter (that is, after steps 31 to 33), the amount of residual liquid and the first residual amount may be compared with each other (that is, in step 1A and step 2A), and the length of the puff interval determination threshold (first period) may be set in accordance with the result of comparison.

Fig. 14B is a flowchart for explaining other example control 1 in Embodiment 6. In Fig. 14B, a part corresponding to a corresponding part in Fig. 14A is assigned a corresponding reference sign and illustrated.

In Fig. 14B, if a negative result is obtained in step 51 (that is, in the case without preliminary heating), the controller 117 performs step 31, step 32, and step 33 in sequence.

If a negative result is obtained in step 33 (that is, the number of consecutive times is less than or equal to the first number of times), the controller 117 sets the puff interval determination threshold (first period) to the reference value TH22 (step 3).

On the other hand, if a positive result is obtained in step 33 (that is, the number of consecutive times is greater than the first number of times), the controller 117 sets the puff interval determination threshold (first period) to the reference value TH21 longer than the reference value TH22 (step 4).

On the other hand, if a positive result is obtained in step 51 (that is, in the case with preliminary heating), the controller 117 performs step 31A, step 32A, and step 33Ain sequence. Note that steps 31A, 32A, and 33A correspond to steps 31, 32, and 33, respectively.

If a negative result is obtained in step 33A (that is, the number of consecutive times is less than or equal to the first number of times), the controller 117 sets the puff interval determination threshold (first period) to the reference value TH22 (step 3).

On the other hand, if a positive result is obtained in step 33A (that is, the number of consecutive times is greater than the first number of times), the controller 117 computes the amount of residual liquid of the aerosol source stored in the liquid storage 213 (step 1A) and determines whether the amount of residual liquid is smaller than the first residual amount (step 2A).

If a negative result is obtained in step 2A (that is, the amount of residual liquid is larger than the first residual amount), the controller 117 sets the puff interval determination threshold (first period) to the reference value TH23 longer than the reference value TH22 (step 61).

On the other hand, if a positive result is obtained in step 2A (that is, the amount of residual liquid is smaller than the first residual amount), the controller 117 sets the puff interval determination threshold (first period) to the longer reference value TH24 in a step-by-step manner as the number of times a short puff interval consecutively appears is greater (step 62).

After setting the determination threshold (first period) in step 3, step 4, step 61, or step 62, the controller 117 performs step 5 to step 11 and step 1100 in sequence and ends one cycle of inhalation.

Although the flow proceeds to step 3 if a negative result is obtained in step 33AinFig. 14B, another reference value may be set.

Fig. 14C is a flowchart for explaining other example control 2 in Embodiment 6. In Fig. 14C, a part corresponding to a corresponding part in Fig. 14B is assigned a corresponding reference sign and illustrated.

In Fig. 14C, if a negative result is obtained in step 33A, the controller 117 sets the puff interval determination threshold (second threshold) to a reference value TH22A longer than the reference value TH22 (step 63). Note that the reference value TH22A is a time shorter than the reference value TH23.

In this embodiment as well, the determination threshold (first period) in the case where a negative result is obtained in step 2A illustrated in Fig. 14A may be shorter than the determination threshold (first period) in the case where a negative result is obtained in step 2.

In this embodiment, in the case where a negative result is obtained in step 2A illustrated in Fig. 14A as well, the controller 117 may perform the processes in steps 31 to 33, and in the case where a positive result is obtained in step 33, the puff interval determination threshold (first period) may be made longer than in the case where a negative result is obtained in step 33.

Although the amount of residual liquid is compared with the first residual amount in step 2 and step 2A in this embodiment, different residual amounts may be the comparison target in step 2 and step 2A. That is, different values may be used as the first residual amount. For example, in the case of using preliminary heating, the first residual amount may be smaller than in the case of not using preliminary heating.

### <Embodiment 7>

In this embodiment, a case will be described where in the case of using preliminary heating and when the amount of residual liquid is small, the length of the determination threshold is variably set in accordance with the length of the puff interval of the immediately preceding instance.

In other words, this embodiment corresponds to an example combination of Embodiment 4 and Embodiment 5.

Note that the other configurations of the aerosol generation device 1 (see Fig. 1) in this embodiment are the same as in Embodiment 1. That is, the external configuration and the internal configuration of the aerosol generation device 1 are the same as in Embodiment 1.

Fig. 15 is a flowchart for explaining example control of the real heating time by the controller 117 (see Fig. 2) used in Embodiment 7. In Fig. 15, a part corresponding to a corresponding part in Fig. 3, Fig. 10, and Fig. 13 is assigned a corresponding reference sign and illustrated. The control by the controller 117 is implemented through execution of a program.

In this embodiment as well, the controller 117 first determines whether preliminary heating is used (step 51).

If a negative result is obtained in step 51, the controller 117 performs operations similar to those in Embodiment 4.

That is, the controller 117 sets the puff interval determination threshold and the real heating time in accordance with the flowchart illustrated in Fig. 10.

Specifically, the controller 117 computes the amount of residual liquid of the aerosol source stored in the liquid storage 213 (see Fig. 2) (step 1), obtains a negative result in step 2 if the amount of residual liquid is larger than the first residual amount and performs step 3, or obtains a positive result in step 2 if the amount of residual liquid is smaller than the first residual amount and performs step 41. That is, when the amount of residual liquid is small in a state in which the function of preliminary heating is OFF, the controller 117 sets the puff interval determination threshold (first period) to the reference value TH24 longer than the puff interval of the immediately preceding instance so that a short puff determination is more likely to be made at the time of setting the real heating time. When a short puff determination is made, the real heating time is set to a time shorter than the reference time LT1, and therefore, the possibility of drying up is accordingly decreased.

Although the amount of residual liquid is computed by calculation in this embodiment, the amount of residual liquid can be obtained by using the amount-of-residual-liquid sensor 113A as in Embodiment 2.

On the other hand, if a positive result is obtained in step 51, the controller 117 performs the puff interval determination threshold and the real heating time in accordance with the flowchart illustrated in Fig. 13.

That is, the controller 117 computes the amount of residual liquid of the aerosol source stored in the liquid storage 213 (step 1A), obtains a negative result in step 2A if the amount of residual liquid is larger than the first residual amount and performs step 3, or obtains a positive result in step 2A if the amount of residual liquid is smaller than the first residual amount and performs step 52A. Note that the value TH23 of the determination threshold set in step 52A is set to a value smaller than the value TH24 set in step 41.

This embodiment attaches importance to the fact that even when the amount of residual liquid is small, in the case of using preliminary heating, the supplied amount of the aerosol source becomes larger than in the case of not using preliminary heating.

Even in the case of using preliminary heating, when the amount of residual liquid becomes small, the possibility of drying up becomes higher than in the case where the amount of residual liquid is large, and therefore, the process in step 41 may be performed instead of step 52A. That is, when the amount of residual liquid is smaller than the first residual amount, a value longer than the puff interval of the immediately preceding instance may be set as the puff interval determination threshold (first period) both in the case of using preliminary heating and in the case of not using preliminary heating.

### <Embodiment 8>

In this embodiment, a case of control of making the real heating time in the case where the preliminary heating mode is ON shorter than the real heating time in the case where the preliminary heating mode is OFF will be described.

Note that the other configurations of the aerosol generation device 1 (see Fig. 1) in this embodiment are the same as in Embodiment 1. That is, the external configuration and the internal configuration of the aerosol generation device 1 are the same as in Embodiment 1.

Fig. 16 is a flowchart for explaining example control of the real heating time by the controller 117 (see Fig. 2) used in Embodiment 8. In Fig. 16, a part corresponding to a corresponding part in Fig. 3 and Fig. 13 is assigned a corresponding reference sign and illustrated. The control by the controller 117 is implemented through execution of a program.

In this embodiment, steps 1, 2, and 3 or steps 1, 2, and 4 described in Embodiment 1 are performed prior to step 51 in which a determination as to whether preliminary heating is used is made.

That is, in this embodiment, the puff interval determination threshold (first period) is set, and thereafter, determination as to whether to use the preliminary heating is made.

To set the puff interval determination threshold (first period), any of Embodiments 2 to 7 described above may be used.

If a negative result is obtained in step 51, the controller 117 determines whether the start of inhalation is detected by the puff sensor 112 (step 5).

If the start of inhalation of an aerosol by the user is not detected, the controller 117 obtains a negative result in step 5. As long as a negative result is obtained in step 5, the controller 117 repeats the determination in step 5.

On the other hand, if the start of inhalation of an aerosol by the user is detected, the controller 117 obtains a positive result in step 5. If a positive result is obtained in step 5, the controller 117 starts real heating (step 1100), and thereafter, obtains the immediately preceding puff interval (step 6).

After obtaining the puff interval, the controller 117 determines whether the puff interval is shorter than the first period (step 7).

If the puff interval is longer than or equal to the first period, the controller 117 obtains a negative result in step 7. In this case, the controller 117 sets the real heating time of the present instance to the reference time LT1 (step 8).

On the other hand, if the puff interval is shorter than the first period, the controller 117 obtains a positive result in step 7. In this case, the controller 117 sets the real heating time of the present instance to the time LT2 shorter than the reference time LT1 (step 9).

After setting the real heating time in step 8 or step 9, the controller 117 performs the processes in step 10 and step 11 in sequence and ends one cycle of inhalation.

The processing operations described above, that is, the processing operations when a negative result is obtained in step 51, are the same as in Embodiment 1.

On the other hand, if a positive result is obtained in step 51, the following process is performed.

First, the controller 117 determines whether the start of inhalation is detected by the puff sensor 112 (step 5A). As long as a negative result is obtained in step 5A, the controller 117 repeats the determination in step 5A, and if a positive result is obtained in step 5A, starts real heating after the end of preliminary heating (step 1100A), and thereafter, obtains the immediately preceding puff interval (step 6A).

After obtaining the puff interval, the controller 117 determines whether the puff interval is shorter than the first period (step 7A), and if the puff interval is greater than or equal to the first period (if a negative result is obtained in step 7A), sets the real heating time of the present instance to the time LT2 shorter than the reference time LT1 (step 9). Note that the threshold used in determination in step 7A may be different from that used in step 7. For example, the threshold used in determination in step 7A may be smaller than the threshold used in determination in step 7. The real heating time when a negative result is obtained in step 7A needs to be shorter than the reference time LT1 and need not be equal to LT2.

On the other hand, if the puff interval is shorter than the first period (if a positive result is obtained in step 7A), the controller 117 sets the real heating time of the present instance to a time LT3 shorter than the reference time LT1 (step 71). Note that the time LT3 is a value shorter than the time LT2. That is, when the puff interval is short (that is, in the case of a short puff), the controller 117 sets the real heating time to a value shorter than in the case where the preliminary heating mode is OFF

In the case where the preliminary heating mode is ON although the length of the real heating time is short, as described with reference to Figs. 11A and 11B, the time taken for the temperature of the aerosol source to reach the boiling point is shorter than in the case where the preliminary heating mode is OFF, and therefore, a substantial difference in the real heating time is less than a set length.

The liquid sending speed in the case where the preliminary heating mode is ON is higher than the liquid sending speed in the case where the preliminary heating mode is OFF, and therefore, more aerosol can be generated in the case where the preliminary heating mode is ON than in the case where the preliminary heating mode is OFF as long as the real heating time is the same.

Even when the time LT3 set in step 71 is shorter than the time LT2 set in step 9, an excessive decrease in the amount of aerosol generated in the real heating time can be avoided.

Compared to a case where the real heating time in the case where the preliminary heating mode is ON and the real heating time in the case where the preliminary heating mode is OFF are the same, power consumption can be reduced, and the hours of use of the aerosol generation device 1 can be extended.

After setting the real heating time in step 71, the controller 117 performs the processes in step 10 and step 11 in sequence and ends one cycle of inhalation.

### <Embodiment 9>

In this embodiment, a control operation when overheating is detected during the real heating time will be described. In this embodiment as well, the external configuration of the aerosol generation device 1 is the same as in Embodiment 1.

Fig. 17 is a diagram schematically illustrating an internal configuration of the aerosol generation device 1 assumed in Embodiment 9. In Fig. 17, a part corresponding to a corresponding part in Fig. 2 is assigned a corresponding reference sign and illustrated.

The aerosol generation device 1 illustrated in Fig. 17 is different from that in Embodiment 1 in that the aerosol generation device 1 illustrated in Fig. 17 includes a coil temperature sensor 113B. The coil temperature sensor 113B is, for example, a thermistor and is disposed in the vicinity of the heater 211, which is formed of a coil. The coil temperature sensor 113B is an example of a second sensor.

Instead of the coil temperature sensor 113B, the current value of a current flowing through the heater 211 or a voltage appearing at a resistor connected in series to the heater 211 may be used to measure the temperature of the heater 211.

A measure against overheating described in this embodiment can be combined with any of Embodiments 1 to 7 described above.

Fig. 18 is a flowchart for explaining example control of the real heating time by the controller 117 (see Fig. 2) used in Embodiment 9. In Fig. 18, a part corresponding to a corresponding part in Fig. 3 is assigned a corresponding reference sign and illustrated. The control by the controller 117 is implemented through execution of a program.

The controller 117 in this embodiment determines whether the start of inhalation is detected by the puff sensor 112 (step 5).

As long as a negative result is obtained in step 5, the controller 117 repeats the determination in step 5.

If a positive result is obtained in step 5, the controller 117 starts real heating (step 1100), and thereafter, obtains the temperature of the coil at the start of inhalation (step 81). That is, the temperature of the heater 211 (see Fig. 2) is obtained.

After obtaining the temperature of the coil, the controller 117 determines whether the temperature of the coil at the start of inhalation is higher than a third temperature (step 82). The third temperature is a determination threshold for overheating.

If the obtained temperature is higher than the third temperature, the controller 117 obtains a positive result in step 82. In this case, the controller 117 forcibly terminates the real heating (step 83). That is, even when the set real heating time does not yet expire, the controller 117 terminates supply of electric power to the heater 211.

Even when supply of electric power is terminated, the temperature of the heater 211 remains high for a while. Therefore, the aerosol is continuously generated for a while.

When heating is terminated before the set real heating time expires, a cooling time before the next instance of inhalation can be extended compared to a case where heating continues until the real heating time expires. As a result, the liquid temperature of the aerosol source at the time of the start of the next instance of inhalation is more likely to be lower than in a case where the control according to this embodiment is not adopted. When overheating is dissipated, the use of the aerosol generation device 1 within a designed temperature can be continued.

On the other hand, if a negative result is obtained in step 82, the controller 117 continues heating in accordance with the set real heating time (step 84).

### <Embodiment 10>

In this embodiment, another control operation when overheating is detected during the real heating time will be described. In this embodiment as well, the external configuration of the aerosol generation device 1 is the same as in Embodiment 1.

Fig. 19 is a diagram schematically illustrating an internal configuration of the aerosol generation device 1 assumed in Embodiment 10. In Fig. 19, a part corresponding to a corresponding part in Fig. 2 is assigned a corresponding reference sign and illustrated.

The aerosol generation device 1 illustrated in Fig. 19 is different from that in Embodiment 1 in that the aerosol generation device 1 illustrated in Fig. 19 includes a liquid temperature sensor 113 C. The liquid temperature sensor 113C measures the temperature of the liquid guide 212 as a target. Therefore, the liquid temperature sensor 113C is disposed in the vicinity of the liquid guide 212. As the liquid temperature sensor 113C, for example, a temperature sensor or a thermistor is used. The liquid temperature sensor 113 C is an example of a third sensor.

A measure against overheating described in this embodiment can be combined with any of Embodiments 1 to 7 described above.

Fig. 20 is a flowchart for explaining example control of the real heating time by the controller 117 (see Fig. 2) used in Embodiment 10. In Fig. 20, a part corresponding to a corresponding part in Fig. 3 is assigned a corresponding reference sign and illustrated. The control by the controller 117 is implemented through execution of a program.

The controller 117 in this embodiment determines as well, whether the start of inhalation is detected by the puff sensor 112 (step 5).

As long as a negative result is obtained in step 5, the controller 117 repeats the determination in step 5.

If a positive result is obtained in step 5, the controller 117 starts real heating (step 1100), and thereafter, obtains the liquid temperature at the start of inhalation (step 91). The liquid temperature here is the temperature of the liquid guide 212.

After obtaining the liquid temperature, the controller 117 determines whether the liquid temperature at the start of inhalation is higher than a fourth temperature (step 92). The fourth temperature is a determination threshold for overheating.

If the obtained liquid temperature is higher than the fourth temperature, the controller 117 obtains a positive result in step 92. In this case, the controller 117 forcibly terminates the real heating (step 93). That is, even when the set real heating time does not yet expire, the controller 117 terminates supply of electric power to the heater 211.

Even when supply of electric power is terminated, the temperature of the heater 211 remains high for a while. Therefore, the aerosol is continuously generated for a while.

When heating is terminated before the set real heating time expires, a cooling time before the next instance of inhalation can be extended compared to a case where heating continues until the real heating time expires. As a result, the liquid temperature of the aerosol source at the time of the start of the next instance of inhalation is more likely to be lower than in a case where the control according to this embodiment is not adopted. When overheating is dissipated, the use of the aerosol generation device 1 within a designed temperature can be continued.

On the other hand, if a negative result is obtained in step 92, the controller 117 continues heating in accordance with the set real heating time (step 94).

### <Embodiment 11>

A form will be described in which when a short puff is detected by using the puff interval determination threshold (first period) set with the technique described in Embodiments 1 to 7, the occurrence of drying up is suppressed not by shortening the real heating time but by setting the voltage value or the current value of a voltage or a current applied to the heater 211 to a low value.

The other configurations of the aerosol generation device 1 (see Fig. 1) in this embodiment are the same as in Embodiment 1. That is, the external configuration and the internal configuration of the aerosol generation device 1 are the same as in Embodiment 1.

Fig. 21 is a flowchart for explaining example control of the real heating time by the controller 117 (see Fig. 2) used in Embodiment 11. In Fig. 21, a part corresponding to a corresponding part in Fig. 3 is assigned a corresponding reference sign and illustrated. The control by the controller 117 is implemented through execution of a program.

First, the controller 117 performs steps 1 to 6 and step 1100 and sets the puff interval determination threshold (first period). As described above, to set the puff interval determination threshold (first period), the technique in any of Embodiments 2 to 7 may be adopted.

After completion of setting of the puff interval determination threshold (first period), the controller 117 determines whether the puff interval is shorter than the first period (step 7). That is, the controller 117 determines whether the latest puff interval is a short puff.

If a negative result is obtained in step 7, the controller 117 sets the maximum voltage value of a voltage that is applied in the real heating time of the present instance to a reference voltage value (step 101). The reference voltage value here is the same as the voltage value used in, for example, Embodiment 1. The reference voltage value here is an example of a second maximum voltage value. Note that current values can be specified as described above.

If a positive result is obtained in step 7, the controller 117 sets the maximum voltage value of the voltage that is applied in the real heating time of the present instance to a value smaller than the reference voltage value (step 102).

That is, the maximum voltage value is set to a low value without shortening the real heating time. The maximum voltage value set in step 102 is an example of a first maximum voltage value. As a result, electric power supplied to the heater 211 in the real heating time becomes smaller than in a case where the puff interval is not short. That is, the electric power becomes smaller than a reference value. As the maximum voltage value is set to a value lower than the reference voltage value, electric power supplied to the heater 211 in the real heating time becomes smaller. As a matter of course, current values can be specified instead of the voltage values.

### <Embodiment 12>

This embodiment assumes a case where the ambient temperature in the environment in which the aerosol generation device 1 is used is low. In countries or regions in high latitudes, the outside air temperature in winter is low. When the outside air temperature is low, the liquid temperature of the aerosol source stored in the liquid storage 213 of the aerosol generation device 1 is low as well, and the viscosity increases simultaneously. When the viscosity increases, the speed of sending the aerosol becomes lower than in a case where the ambient temperature is high even in the case where the puff interval is long as well as in the case where the puff interval is short. As a result, when the supplied amount of the aerosol source supplied to the heater 211 before the start of inhalation drops below a liquid amount necessary for generation of an aerosol, an event the same as drying up occurs.

Accordingly, this embodiment pays attention to the ambient temperature in the environment or atmosphere in which the aerosol generation device 1 is used.

In this embodiment as well, the external configuration of the aerosol generation device 1 is the same as in Embodiment 1. However, the internal configuration of the aerosol generation device 1 assumed in this embodiment is different from that in Embodiment 1 in part.

Fig. 22 is a diagram schematically illustrating an internal configuration of the aerosol generation device 1 assumed in Embodiment 12. In Fig. 22, a part corresponding to a corresponding part in Fig. 2 is assigned a corresponding reference sign and illustrated.

The aerosol generation device 1 illustrated in Fig. 22 is different from the aerosol generation device 1 illustrated in Fig. 2 in that the aerosol generation device 1 illustrated in Fig. 22 is provided with an ambient temperature sensor 113D. The ambient temperature sensor 113D measures the ambient temperature as a target. Therefore, it is desirable to dispose the ambient temperature sensor 113D so as to be spaced apart from the heat source in the device as much as possible. The viscosity of the aerosol source depends on the liquid temperature of the aerosol source stored in the liquid storage 213, and therefore, the liquid temperature sensor 113C (see Fig. 19) may be disposed in the vicinity of the liquid storage 213.

Fig. 23 is a flowchart for explaining example control of the real heating time by the controller 117 (see Fig. 2) used in Embodiment 12. In Fig. 23, a part corresponding to a corresponding part in Fig. 3 is assigned a corresponding reference sign and illustrated. The control by the controller 117 is implemented through execution of a program.

First, the controller 117 performs steps 1, 2, and 3 or steps 1, 2, and 4 and sets the puff interval determination threshold (first period). As described above, to set the puff interval determination threshold (first period), the technique in any of Embodiments 2 to 7 may be adopted.

Subsequently, the controller 117 determines whether the start of inhalation is detected by the puff sensor 112 (step 5). This determination is made when real heating is started in response to the start of inhalation by the user.

If the start of inhalation of an aerosol by the user is not detected, the controller 117 obtains a negative result in step 5. As long as a negative result is obtained in step 5, the controller 117 repeats the determination in step 5.

On the other hand, if the start of inhalation of an aerosol by the user is detected, the controller 117 obtains a positive result in step 5. If a positive result is obtained in step 5, the controller 117 starts real heating (step 1100), and thereafter, obtains the ambient temperature at the start of inhalation (step 111). The ambient temperature is the ambient temperature around the aerosol generation device 1.

After obtaining the ambient temperature, the controller 117 determines whether the ambient temperature at the start of inhalation is lower than a threshold for ambient temperature determination (hereinafter referred to as "ambient temperature threshold") (step 112). The ambient temperature threshold is determined in accordance a relationship between the viscosity of the aerosol source and the ambient temperature.

If the ambient temperature is higher than or equal to the ambient temperature threshold, the controller 117 obtains a negative result in step 112. In this case, the controller 117 sets the real heating time of the present instance to the reference time LT1 (step 8).

On the other hand, if the ambient temperature is lower than the ambient temperature threshold, the controller 117 obtains a positive result in step 112. In this case, the controller 117 sets the real heating time of the present instance to the time LT2 shorter than the reference time LT1 (step 9).

After setting the real heating time in step 8 or step 9, the controller 117 performs step 10 and step 11 in sequence and ends one cycle of inhalation.

In this embodiment, the controller 117 pays attention to the ambient temperature at which the efficiency of aerosol generation decreases, and detects use in an environment in which drying up occurs. Therefore, the occurrence of drying up can be effectively suppressed.

Figs. 24A to 24C are diagrams for explaining a relationship between a puff interval and a setting of the real heating time in Embodiment 12. Fig. 24A illustrates example timings of inhalation (puffs), Fig. 24B illustrates changes in the ambient temperature, and Fig. 24C illustrates example settings of the real heating time. In Figs. 24A to24C, a part corresponding to a corresponding part in Figs. 5A to 5C is assigned a corresponding reference sign and illustrated. The vertical axis in Fig. 24A represents the puff intensity, the vertical axis in Fig. 24B represents the ambient temperature, and the vertical axis in Fig. 24C represents the intensity of heating. The horizontal axis in Figs. 24A to 24C represents time.

Both Figs. 24(A) and 24(C) illustrate a case where the start of heating by the heater 211 and the start of inhalation by the user coincide with each other. Fig. 24B illustrates changes in the ambient temperature in the environment in which the aerosol generation device 1 is used. Fig. 24B assumes a scene in which the user moves from a heated room to outdoors in winter, which results in a drop, in the ambient temperature, that affects the viscosity of the aerosol source.

In Fig. 24A as well, the number of instances of inhalation (puff) is five. However, in Fig. 24A, none of the interval between the first puff and the second puff, the interval between the second puff and the third puff, the interval between the third puff and the fourth puff, and the interval between the fourth puff and the fifth puff is a short puff.

Note that the first puff, the second puff, and the third puff are performed indoors, and the fourth puff and the fifth puff are performed outdoors. Therefore, the ambient temperature drops between the third puff and the fourth puff in Fig. 24B.

It is assumed that between the third puff and the fourth puff, there is a time in which the liquid temperature of the aerosol source drops, and consequently, the liquid temperature of the aerosol source becomes close to the ambient temperature at the start of the fourth puff. It is also assumed that the liquid temperature of the aerosol source at this time drops to a value lower than the ambient temperature threshold. Therefore, in the example illustrated in Fig. 24C, the real heating times of the first puff, the second puff, and the third puff are set to the reference time LT1 while the real heating times of the fourth puff and the fifth puff are set to the time LT2 shorter than the reference time LT1.

As a result, in the fourth puff and the fifth puff, even when the supplied amount of the aerosol source supplied to the heater 211 before the start of inhalation is small because of the low ambient temperature, the real heating time is made shorter than the reference time LT2, and therefore, drying up does not occur.

### <Embodiment 13>

Although the aerosol generation device 1 having the power supply button 11 (see Fig. 1) has been described in the above-described embodiments, the present invention is applicable to the aerosol generation device 1 that does not have the power supply button 11.

Fig. 25 is a diagram for explaining an example external configuration of the aerosol generation device 1 assumed in Embodiment 13. In Fig. 25, a part corresponding to a corresponding part in Fig. 1 is assigned a corresponding reference sign and illustrated.

In this embodiment, in response to detection of the start of inhalation by the user, supply of electric power to the heater 211 (see Fig. 2) is started.

### <Embodiment 14>

In this embodiment, the aerosol generation device 1 having a mechanism for heating a substrate that includes an aerosol in addition to the mechanism for heating the aerosol source, which is a liquid, will be described.

Fig. 26 is a diagram schematically illustrating an example internal configuration of the aerosol generation device 1 assumed in Embodiment 14. In Fig. 26, a part corresponding to a corresponding part in Fig. 2 is assigned a corresponding reference sign and illustrated.

The aerosol generation device 1 illustrated in Fig. 26 is provided with a holder 301 used to hold a stick substrate 400, a heater 302 disposed around the outer circumference of the holder 301, and a heat insulator 303 disposed around the outer circumference of the heater 302 in addition to the power supply 111, the puff sensor 112, the power supply button sensor 113, the notifier 114, the memory 115, the communicator 116, the controller 117, the heater 211, the liquid guide 212, and the liquid storage 213.

Fig. 26 illustrates a state in which the stick substrate 400 is mounted in the holder 301. The user performs an inhalation action in a state in which the stick substrate 400 is inserted into the holder 301.

In the aerosol generation device 1, the airflow path 40 for conveying air flowing in through the air inlet hole 21 to a bottom 301C of the holder 301 via the liquid guide 212 is formed. Therefore, in response to an inhalation action by the user, air flowing in through the air inlet hole 21 flows through the inside of the airflow path 40 along the arrow 500. This flow of air is mixed with an aerosol generated at the heater 211 and an aerosol generated at the heater 302.

Note that the controller 117 in this embodiment controls a heating operation of the heater 302 as well as a heating operation of the heater 211. At this time, the controller 117 obtains information about, for example, the temperature of the heater 302 with a sensor not illustrated.

The holder 301 has a substantially cylindrical form. Therefore, the inside of the holder 301 is a hollow. This hollow is referred to as an internal space 301A. The internal space 301A has a diameter substantially the same as that of the stick substrate 400 and accommodates the stick substrate 400 inserted through an opening 301B in a state in which the leading end of the stick substrate 400 is in contact. That is, the stick substrate 400 is held in the internal space 301A.

The holder 301 has the bottom 301C on a side opposite to the opening 301B. The bottom 301C is coupled to the airflow path 40.

The inside diameter of the holder 301 is made smaller than the outside diameter of the stick substrate 400 in at least a part of the tubular body in the height direction. Therefore, the outer circumference surface of the stick substrate 400 inserted into the internal space 301A through the opening 301B is under pressure by the internal wall of the holder 301. With this pressure, the stick substrate 400 is held by the holder 301.

The holder 301 also has a function of defining the flow path of air passing through the stick substrate 400. Here, the bottom 301C is an inlet hole for air into the holder 301, and the opening 301B is an outlet hole for air from the holder 301.

The stick substrate 400 is a member having a substantially cylindrical form. The stick substrate 400 assumed in this embodiment is constituted by a substrate 401 and an inhalation port 402.

The substrate 401 accommodates an aerosol source. The aerosol source is a substance that is atomized when heated to generate an aerosol. The aerosol source accommodated in the substrate 401 is, for example, a substance derived from tobacco, such as a processed material obtained by forming shredded tobacco or a tobacco raw material into a granular form, a sheet form, or a powder form. However, the aerosol source accommodated in the substrate 401 may include a substance not derived from tobacco but made of a plant (examples of which include a mint and a herb) other than tobacco. For example, the aerosol source may include a flavor component, such as menthol.

When the aerosol generation device 1 is a medical inhaler, the aerosol source of the stick substrate 400 may include medicine to be inhaled by a patient. Note that the aerosol source is not limited to a solid and may be a liquid, such as polyhydric alcohol, which is, for example, glycerine or propylene glycol, or water.

At least a part of the substrate 401 is accommodated in the internal space 301A of the holder 301 in a state in which the stick substrate 400 is held by the holder 301.

The inhalation port 402 is a member that is held in the user's mouth during inhalation. At least a part of the inhalation port 402 protrudes from the opening 301B in the state in which the stick substrate 400 is held by the holder 301.

When the user holds the inhalation port 402 protruding from the opening 301B in their mouth and inhales, air flows into the bottom 301C of the holder 301 through the air inlet hole 21 as described above. The flowing-in air passes through the internal space 301A of the holder 301 and the substrate 401 and reaches the inside of the user's mouth. Note that the gas that passes through the internal space 301A of the holder 301 and the substrate 401 is mixed with an aerosol generated from the substrate 401.

The heater 302 heats the aerosol source included in the substrate 401 to thereby atomize the aerosol source and generate an aerosol. The heater 302 is formed of any material, such as metal or a polyimide. For example, the heater 302 is formed in a film form and is disposed so as to cover the outer circumference of the holder 301.

When the heater 302 produces heat, the aerosol source included in the stick substrate 400 is heated from the outer circumference of the stick substrate 400 and atomized to thereby generate an aerosol.

The heater 302 produces heat in response to supply of electric power from the power supply 111. For example, when specific user input is detected by, for example, a sensor not illustrated, supply of electric power to the heater 302 starts and an aerosol is generated.

When the temperature of the stick substrate 400 reaches a specific temperature in response to heating by the heater 302, generation of an aerosol starts, and inhalation by the user becomes possible.

Thereafter, when specific user input is detected by, for example, a sensor not illustrated, supply of electric power to the heater 302 stops.

While inhalation by the user is detected by the puff sensor 112, supply of electric power to the heater 302 may continue and an aerosol may be kept generated.

### <Other Embodiments>

Although embodiments of the present invention have been described above, the technical scope of the present invention is not limited to the scope described in the embodiments. It is obvious from the description in the claims that an embodiment obtained by making various changes or improvements to the above-described embodiments is included in the technical scope of the present invention.

For example, although the puff interval setting threshold (first period) is set, and thereafter, the start of inhalation is detected in, for example, Embodiment 1 described above, it is possible to detect the start of inhalation, and thereafter, set the puff interval setting threshold (first period).

For example, although a case where the user can choose whether to use the preliminary heating mode has been described in, for example, Embodiment 5 described above, the aerosol generation device 1 may be used while the preliminary heating mode is always kept ON.

### Reference Signs List

- 1: aerosol generation device
- 10: power supply unit
- 11: power supply button
- 20, 30: cartridge
- 21: air inlet hole
- 40: airflow path
- 42: air outlet hole
- 112: puff sensor
- 113: power supply button sensor
- 113A: amount-of-residual-liquid sensor
- 113B: coil temperature sensor
- 113C: liquid temperature sensor
- 113D: ambient temperature sensor
- 117: controller
- 211, 302: heater
- 212: liquid guide
- 213: liquid storage

## Claims

1. A circuit unit for an aerosol generation device, comprising:
a controller that controls supply of electric power to a load that heats an aerosol source, wherein
when a residual amount of the aerosol source is smaller than a first residual amount, the controller sets a first period that is used to determine whether a length of an interval between a preceding instance of aerosol inhalation and a present instance of aerosol inhalation is long or short to a value longer than a reference value.

2. The circuit unit for an aerosol generation device according to claim 1, wherein
the controller computes the residual amount of the aerosol source by calculation and controls a length of the first period on the basis of the computed residual amount.

3. The circuit unit for an aerosol generation device according to claim 1, further comprising:
a first sensor that detects the residual amount of the aerosol source, wherein
the controller controls a length of the first period on the basis of the residual amount detected by the first sensor.

4. The circuit unit for an aerosol generation device according to claim 1, wherein
when a number of times an instance of inhalation whose inter-aerosol-inhalation interval is shorter than the first period consecutively appears exceeds a first number of times, the controller controls the first period that is used in a next instance of inhalation and beyond so as to be longer in a step-by-step manner as the number of times increases.

5. The circuit unit for an aerosol generation device according to claim 1, wherein
when the residual amount of an aerosol is smaller than the first residual amount, the controller controls the first period so as to be a time longer than an interval of an immediately preceding instance.

6. The circuit unit for an aerosol generation device according to claim 1, wherein
when the aerosol source is heated at a second temperature lower than a first temperature prior to heating of the aerosol source at the first temperature, the heating involving aerosol generation, the controller controls the first period that is used when the residual amount of an aerosol is smaller than the first residual amount so as to be a value smaller than the first period that is used only in a case of heating involving aerosol generation and when the residual amount of the aerosol is smaller than the first residual amount.

7. The circuit unit for an aerosol generation device according to claim 6, wherein
when a number of times an instance of inhalation whose inter-aerosol-inhalation interval is shorter than the first period consecutively appears exceeds a first number of times, the controller controls the first period that is used in a next instance of inhalation and beyond so as to be longer in a step-by-step manner as the number of times increases.

8. The circuit unit for an aerosol generation device according to claim 6, wherein
when the residual amount of an aerosol is smaller than the first residual amount, the controller controls the first period so as to be a time longer than an interval of an immediately preceding instance.

9. The circuit unit for an aerosol generation device according to claim 6, wherein
when heating not involving aerosol generation is performed, the controller controls an amount of electric power that is supplied to the load for aerosol generation so as to be a value smaller than an amount of electric power that is supplied to the load when only heating involving aerosol generation is performed.

10. The circuit unit for an aerosol generation device according to any one of claims 1 to 9, further comprising:
a second sensor that detects a temperature of the load, wherein
when the temperature detected by the second sensor reaches a third temperature, the controller forcibly terminates heating by the load.

11. The circuit unit for an aerosol generation device according to any one of claims 1 to 9, wherein
the controller further includes a third sensor that detects a temperature of the aerosol source, and
at a time point when the temperature detected by the third sensor reaches a fourth temperature, the controller forcibly terminates heating by the load.

12. The circuit unit for an aerosol generation device according to any one of claims 1 to 9, wherein
when an inter-aerosol-inhalation interval is shorter than the first period, the controller controls a first maximum voltage value of a voltage that is supplied to the load for aerosol generation so as to be a value smaller than a second maximum voltage value of a voltage that is supplied to the load when the inter-aerosol-inhalation interval is longer than a threshold.

13. An aerosol generation device comprising:
a controller that controls supply of electric power to a load that heats an aerosol source, wherein
when a residual amount of the aerosol source is smaller than a first residual amount, the controller sets a first period that is used to determine whether a length of an interval between a preceding instance of aerosol inhalation and a present instance of aerosol inhalation is long or short to a value longer than a reference value.

14. A program for causing a computer that controls supply of electric power to a load that heats an aerosol source to implement:
a function of, when a residual amount of the aerosol source is smaller than a first residual amount, setting a first period that is used to determine whether a length of an interval between a preceding instance of aerosol inhalation and a present instance of aerosol inhalation is long or short to a value longer than a reference value.
